# EUROPEAN PATENT SPECIFICATION

(11) **EP 1 286 645 B1**
(45) Date of publication and mention of the grant of the patent: **18.10.2006**
(21) Application number: 00939413.1
(22) Date of filing: 30.05.2000
(51) Int. Cl.: A61K 8/81, A61K 8/73, A61K 8/39, A61K 8/86, A61K 8/898, A61Q 5/12

(54) **HAIR CONDITIONING COMPOSITION COMPRISING A FRIZZ CONTROL AGENT**
HAARKONDITIONIERUNGSMITTEL ENTHLATEND EIN DIE KRÄUSELUNG STEUERNDES MITTEL
COMPOSITION DE SOIN CAPILLAIRE CONTENANT UN AGENT DE LISSAGE

(43) Date of publication of application: 05.03.2003
(73) Proprietor: THE PROCTER & GAMBLE COMPANY, Cincinnati, Ohio 45202 (US)
(72) Inventor: SNYDER, Michael, Albert, Kobe 658-0032 (JP); SOMEYA, Kazuyoshi, Utsunomiya 320-0851 (JP)
(74) Representative: Kohol, Sonia
(86) International application number: PCT/US2000/014870
(87) International publication number: WO 2001/091705

(56) References cited:
- EP-A- 0 978 271
- WO-A-00/40212
- WO-A-01/00141
- WO-A-92/17154
- WO-A-95/09600
- US-A- 5 344 643
- US-A- 5 858 340
- US-A- 5 989 532

## Description

### TECHNICAL FIELD

The present invention relates to hair conditioning compositions comprising a frizz control agent and a thickening agent selected from the group consisting of an acrylic acid/alkyl acrylate copolymer, an acrylates copolymer, a crosslinked polymer, and mixtures thereof.

### BACKGROUND

Hair is often subjected to a wide variety of insults that can cause damage. These include shampooing, rinsing, drying, heating, combing, styling, perming, coloring, exposure to the elements, etc. Thus, the hair is often in a dry, rough, lusterless or frizzy condition due to abrasion of the hair surface and removal of the hair's natural oils and other natural conditioning and moisturizing components. Additionally, hair is subjected to weather-related changes, such as changes in humidity, which can leave hair in a frizzy condition.

Frizzy condition of the hair often causes an expanded and unruly hair and makes it difficult to control the hair style. For consumers who desire well aligned hair, such expanded and unruly hair is not desirable. The term "frizz control" as used herein means to control hair frizz, i.e., to reduce frizz condition of the hair or to prevent the hair from causing frizzy condition. For consumers who desire hair volume-down, for example, such consumers whose hair are easy to expand, such consumers having coarse, wavy, curly, or much volume of hair, such expanded and unruly hair is not desirable. The term "hair volume-down" as used herein relates to decreased bulk hair volume or reduced hair expansion.

A variety of approaches have been developed to control hair frizz. These include reactive chemistry approaches aimed at a permanent restructuring of hair, and application of oily leave-on products to weigh down hair. The use of reactive chemistry provides a permanent frizz reduction benefit. However, the reactive chemistry methods and compositions are harsh on the hair structure and can cause hair to split or break and can also result in a loss of hair shine. Skin and/or eye irritation from the relatively harsh chemicals used in reactive chemistry methods is also common.

Typically, leave-on conditioner type hair formulations provide advantages over other more permanent frizz reduction approaches. For example, leave-on formulations are typically less damaging to the hair. Also, leave-on formulations are more convenient because the consumer can use the product at any time and then wash the product out of the hair with one washing. Another benefit is that the product may be applied to parts of the hair most in need of the frizz control benefits.

Commonly, hair conditioning benefits are provided through the use of hair conditioning agents such as cationic surfactants, cationic polymers, silicone conditioning agents, hydrocarbon and other organic oils and solid aliphatics such as fatty alcohols. However, such conditioning agents are often impractical for using in the large amounts necessary for hair frizz control and hair volume-down, Usage of large amounts of conditioning agents that work for hair frizz control and hair volume-down by coating and weighing down the hair commonly results in a poor perception of hair cleanliness and hair feel, for example, leaving the hair and hands with a tacky, dirty, feeling.

Based on the foregoing, there remains a desire to provide hair conditioning compositions which provide improved frizz control benefit and hair volume-down benefit while retaining good conditioning benefits and good hair feel and appearance, i.e., which provide improved frizz control benefit and hair volume-down benefit in addition to other conditioning benefits such as smoothness, softness, and reduction of friction, while reducing sticky and greasy feeling.

None of the existing art provides all of the advantages and benefits of the present invention.

US 5, 989, 532 discloses hair treatment compositions for frizz control.

### SUMMARY

The present invention is directed to a hair conditioning composition (for leave-on use on the hair) comprising:
(1) a thickening agent selected from the group consisting of (i), (ii), (iii), and (iv);
   (i) an acrylic acid/alkyl acrylate copolymer having the following formula: 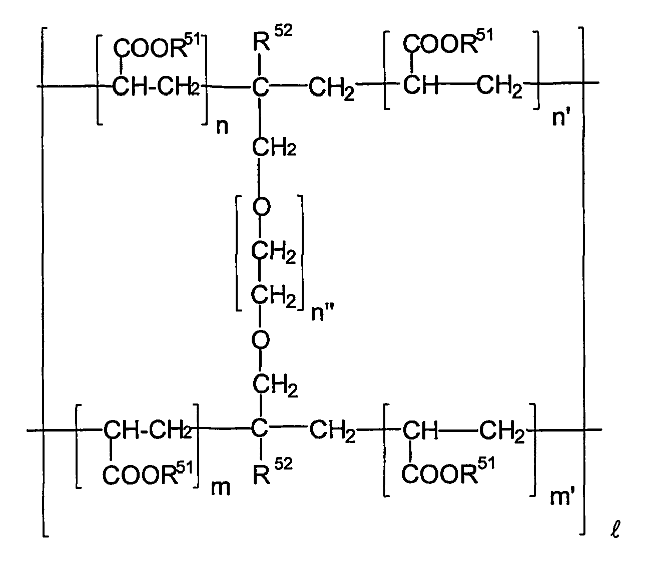 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000;
   (ii) an acrylates copolymer comprising by weight:
      (a) from about 5% to about 80% of an acrylate monomer selected from the group consisting of a C₁-C₆ alkyl ester of acrylic acid, a C₁-C₆ alkyl ester of methacrylic acid, and mixtures thereof;
      (b) from about 5% to about 80% of a monomer selected from the group consisting of a vinyl-substituted heterocyclic compound containing at least one of a nitrogen or sulfur atom, a (meth)acrylamide, a mono- or di-(C₁₋C₄)alkylamino(C₁-C₄)alkyl(meth)acrylate, a mono- or di-(C₁₋C₄)alkylamino(C₁-C₄)alkyl(meth)acrylamide, and mixtures thereof; and
      (c) from 0% to about 30% of an associative monomer;
      (iii) a crosslinked polymer having the formula (A)ₘ(B)ₙ(C)ₚ, wherein:
         (A) is selected from the group consisting of a dialkylaminoalkyl acrylate, a quaternized dialkylaminoalkyl acrylate, an acid addition salt of a quaternized dialkylaminoalkyl acrylate, and mixtures thereof;
         (B) is selected from the group consisting of a dialkylaminoalkyl methacrylate, a quaternized dialkylaminoalkyl methacrylate, an acid addition salt of a quaternized dialkylaminoalkyl methacrylate, and mixtures thereof;
         (C) is a nonionic monomer polymerizable with (A) or (B); and m, n, and p are independently zero or greater, but at least one of m or n is one or greater; and
      (iv) mixtures thereof;
(2) a frizz control agent selected from the group consisting of (i), (ii), and (iii),
   (i) PEG-modified glycerides having the structure: 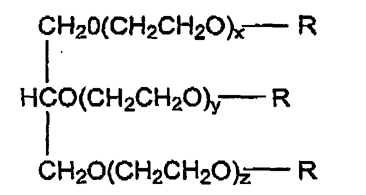 wherein one or more of the R groups is selected from saturated or unsaturated fatty acid moieties derived from animal or vegetable oils wherein the fatty acid moieties have a carbon length chain of from 12 and 22, any other R groups are hydrogen, x, y, z are independently zero or more, the average sum of x+y+z is equal to from about 10 to about 45;
   (ii) PEG-modified glyceryl fatty acid esters having the structure: 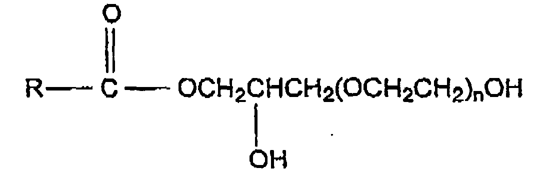 wherein R is an aliphatic group having from 12 to 22 carbon chain length, and n has an average value of from 5 to 40;
   (iii) and mixtures thereof;
(3) an aqueous carrier.

These and other features, aspects, and advantages of the present invention will become evident to those skilled in the art from a reading of the present disclosure.

### DETAILED DESCRIPTION

While the specification concludes with claims which particularly point out and distinctly claim the invention, it is believed the present invention will be better understood from the following description.

Citation of any reference is not an admission regarding any determination as to its availability as prior art to the claimed invention.

Herein, "comprising" means that other steps and other ingredients which do not affect the end result can be added. This term encompasses the terms "consisting of" and "consisting essentially of'.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

The aspects and embodiments of the present invention set forth in this document have many advantages. For example, the hair conditioning compositions of the present invention provide improved frizz control benefit and hair volume-down benefit while retaining good conditioning benefits and good hair feel and appearance, i.e., which provide improved frizz control benefit and hair volume-down benefit in addition to other conditioning benefits such as smoothness, softness, and reduction of friction, and leave the hair and hands with a clean feeling.

### THICKENING AGENT

The compositions of the present invention comprises a thickening agent selected from the group consisting of an acrylic acid/alkyl acrylate copolymer, an acrylates copolymer, a crosslinked polymer, and mixtures thereof. In one preferred embodiment, the composition of the present invention comprises the acrylic acid/alkyl acrylate copolymer. In another preferred embodiment, the composition of the present invention comprises a thickening system which comprises at least 2 thickening agents selected from the group consisting of a hydrophobically modified cellulose ether, the acrylates copolymer, and the crosslinked polymer. The thickening agent useful herein is believed to provide improved conditioning benefits to the hair such as smoothness, softness, and reduction of friction, be easy to apply on the hair, and leave the hair and hands with a clean feeling.

The thickening agent useful herein can also provide appropriate viscosity and rheology properties to the composition, so that the composition of the present composition has a suitable viscosity, preferably from about 1,000 mPa.s to about 100,000 mPa·s, more preferably from about 2,000 mPa·s to about 50,000 mPa·s. The viscosity herein can be suitably measured by Brookfield RVT at 20rpm at 20°C using either spindle #4, 5, 6 or 7 depending on the viscosity and the characteristic of the composition.

The thickening agent can be included in the composition of the present invention at a level by weight of preferably from about 0.05% to about 10%, more preferably from about 0.1 % to about 8%, still preferably from about 0.1 % to about 5%.

### Acrylic acid/alkyl acrylate copolymer

The compositions of the present invention may comprise an acrylic acid/alkyl acrylate copolymer as a thickening agent. The acrylic acid/alkyl acrylate copolymers useful herein have the following formula: 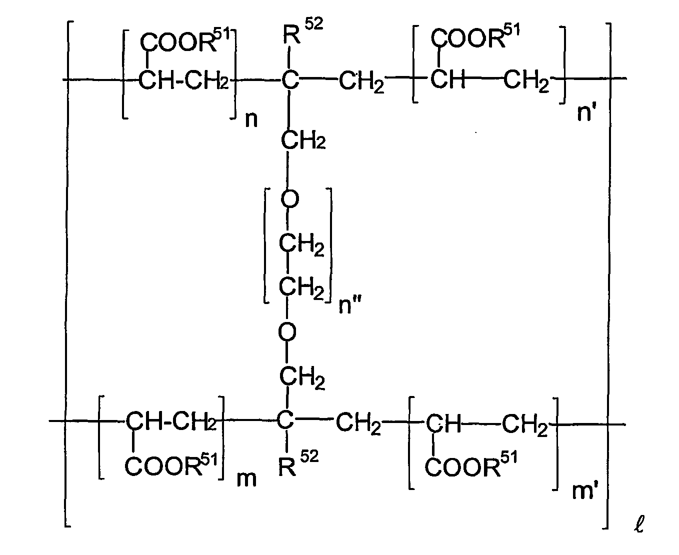 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000.

Commercially available acrylic acid/alkyl acrylate copolymers useful herein include: CTFA name Acrylates/C10-30 Alkyl Acrylate Crosspolymer having tradenames Pemulen TR-1, Pemulen TR-2, Carbopol 1342, Carbopol 1382, and Carbopol ETD 2020, all available from B. F. Goodrich Company.

Neutralizing agents may be included to neutralize the acrylic acid/alkyl acrylate copolymers herein. Nonlimiting examples of such neutralizing agents include sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof.

### Acrylates copolymer

The compositions of the present invention may comprise an acrylates copolymer as a thickening agent.

The acrylates copolymer useful herein are preferably nonionic or cationic polymers, more preferably cationic polymers especially when the composition of the present invention has an acidic pH. The copolymer useful herein comprises by weight:
(a) from about 5% to about 80% of an acrylate monomer selected from the group consisting of a C₁-C₆ alkyl ester of acrylic acid, a C₁-C₆ alkyl ester of methacrylic acid, and mixtures thereof;
(b) from about 5% to about 80% of a monomer selected from the group consisting of a vinyl-substituted heterocyclic compound containing at least one of a nitrogen or sulfur atom, (meth)acrylamide, a mono- or di-(C₁₋C₄)alkylamino(C₁-C₄)alkyl(meth)acrylate, a mono- or di-(C₁₋C₄)alkylamino(C₁-C₄)alkyl(meth)acrylamide, and mixtures thereof; and
(c) from 0% to about 30% of an associative monomer.

The acrylate monomers (a) are selected from the group consisting of esters prepared from acrylic acid and C₁-C₆ alcohols such as methyl, ethyl, or propyl alcohol, and esters prepared from methacrylic acid and C₁-C₆ alcohols. Preferred are C₂-C₆ alkyl esters of acrylic acid, and more preferred is ethyl acrylate. The acrylate monomers (a) are included in the acrylates copolymer at a level by weight of from about 5% to about 80%, preferably from about 15% to about 70%, and more preferably from about 40% to about 70%.

The monomer (b) are selected from the group consisting of a vinyl-substituted heterocyclic compound containing at least one of a nitrogen or sulfur atom, (meth)acrylamide, a mono- or di-(C₁-C₄)alkylamino(C₁₋C₄)alkyl(meth)acrylate, a mono- or di-(C₁-C₄)alkylamino(C₁₋C₄)alkyl(meth)acrylamide. Preferred are mono- or di-(C₁-C₄)alkylamino(C₁₋C₄)alkyl(meth)acrylates. Exemplary monomers (b) include N,N-dimethylamino ethyl methacrylate (DMAEMA), N,N-diethylamino ethyl acrylate, N,N-diethylamino ethyl methacrylate, N-t-butylamino ethyl acrylate, N-t-butylamino ethyl methacrylate, N,N-dimethylamino propyl acrylamide, N,N-dimethylamino propyl methacrylamide, N,N-diethylamino propyl acrylamide, and N,N-diethylamino propyl methacrylamide.

The monomers (b) are included in the acrylates copolymer at a level by weight of from about 5% to about 80%, preferably from about 10% to about 70%, and more preferably from about 20% to about 60%.

The associative monomers (c) are preferably selected from the group consisting of:
(i) urethane reaction products of a monoethylenically unsaturated isocyanate and nonionic surfactants comprising C₁-C₄ alkoxy-terminated, blockcopolymers of 1,2-butylene oxide and 1,2-ethylene oxide, as disclosed in U.S. patent 5,294,692;
(ii) an ethylenically unsaturated copolymerizable surfactant monomer obtained by condensing a nonionic surfactant with an acid, wherein the acid is selected from the group consisting of an α,β-ethylenically unsaturated carboxylic acid, anhydrides of α,β-ethylenically unsaturated carboxylic acids, and mixtures thereof, preferably, selected from the group consisting of a C₃-C₄ mono- or di-carboxylic acid, anhydrides of C₃-C₄ mono- or di-carboxylic acids, and mixtures thereof, more preferably, selected from the group consisting of acrylic acid, methacrylic acid, crotonic acid, maleic acid, maleic anhydride, itaconic acid, itaconic anhydride, and mixtures thereof, as disclosed in U.S. patent 4,616,074;
(iii) a surfactant monomer selected from the urea reaction product of a monoethylenically unsaturated monoisocyanate with a nonionic surfactant having amine functionality, as disclosed in U.S. patent 5,011,978;
(iv) an allyl ether of the formula: CH₂=CR'CH₂OAₘBₙAₚR, wherein R' is hydrogen or methyl, A is propyleneoxy or butyleneoxy, B is ethyleneoxy, n is zero or an integer, m and p are independently zero or an integer less than n, and R is a hydrophobic group having at least 8 carbon atoms;
(v) a nonionic urethane monomer which is the urethane reaction product of a monohydric nonionic surfactant with a monoethylenically unsaturated isocyanate, preferably one lacking ester groups such as alpha, alpha-dimethyl-m-iso-propenyl benzyl isocyanate as disclosed in U.S. patent Re. 33,156; and
(vi) mixtures thereof.

Such associative monomers (c) include those disclosed in U.S. patent 3,657,175, U.S. patent 4,384,096, U.S. patent 4,616,074, U.S. patent 4,743,698, U.S. patent 4,792,343, U.S. patent 5,011,978, U.S. patent 5,102,936, U.S. patent 5,294,692, U.S. patent Re. 33,156. Particularly preferred associative monomers (c) are those described in above (ii), i.e., the ethylenically unsaturated copolymerizable surfactant monomer obtained by condensing a nonionic surfactant with an acid, wherein the acid is selected from the group consisting of α,β-ethylenically unsaturated carboxylic acids, anhydrides of α,β-ethylenically unsaturated carboxylic acids, and mixtures thereof. More preferred associative monomers (c) are ethylenically unsaturated copolymerizable surfactant monomers obtained by condensing a nonionic surfactant with itaconic acid.

The associative monomers (c) are included in the acrylates copolymer at a level by weight of from 0% to about 30%, preferably from about 0.1% to about 10%.

In addition to required and preferred monomers discussed above, monomers which provide cross-linking in the polymer also may be utilized in relatively low amounts, preferably up to about 2%, more preferably from about 0.1% to about 1.0% by weight, based on the total weight of monomers used to prepare the polymer. Cross-linking monomers include multi-vinyl-substituted aromatic monomers, multi-vinyl-substituted alicyclic monomers, id-functional esters of phthalic acid, di-functional esters of methacrylic acid, multi-functional esters of acrylic acid, N-methylene-bis-acrylamide and multi-vinyl-substituted aliphatic monomers such as dienes, trienes, and tetraenes. Exemplary cross-linking monomers include divinylbenzene, trivinylbenzene, 1,2,4-trivinylcyclohexane, 1,5-hexadiene, 1,5,9-decatriene, 1,9-decadiene, 1,5-heptadiene, di-allyl phthalate, ethylene glycol dimethacrylate, polyethylene glycol dimethacrylate, penta- and tetra-acrylates, triallyl pentaerythritol, octaallyl sucrose, cycloparaffins, cycloolefins and N-methylene-bis-acrylamide. The polyethylene glycol dimethacrylates are preferred in view of thickening benefit particularly in aqueous compositions having an acidic pH.

Commercially available acrylates copolymers useful herein include: Acrylates/Aminoacrylates/C₁₀₋₃₀Alkyl PEG-20 Itaconate copolymer having tradename Structure Plus available from National Starch.

### Crosslinked polymer

The compositions of the present invention may comprise a crosslinked polymer as a thickening agent.

Crosslinked polymers useful herein are generally described in U.S. Patent 5,100,660, U.S. Patent 4,849,484, U.S. Patent 4,835,206, U.S. Patent 4,628,078, U.S. Patent 4,599,379, and EP 228,868.

The crosslinked polymers useful herein are preferably nonionic or cationic polymers, more preferably cationic polymers. The crosslinked polymer useful herein comprises the monomer units and has the formula (A)ₘ(B)ₙ(C)ₚ wherein:
(A) is a dialkylaminoalkyl methacrylate, a quaternized dialkylaminoalkyl methacrylate, an acid addition salt of a quaternized dialkylaminoalkyl methacrylate, or mixtures thereof;
(B) is a dialkylaminoalkyl methacrylate, a quaternized dialkylaminoalkyl methacrylate, an acid addition salt of a quaternized dialkylaminoalkyl methacrylate, or mixtures thereof;
(C) is a nonionic monomer polymerizable with (A) or (B); and
m, n, and p are independently zero or greater, but at least one of m or n is one or greater.

The monomer (C) can be selected from any of the commonly used monomers. Non-limiting examples of these monomers include ethylene, propylene, butylene, isobutylene, eicosene, maleic anhydride, acrylamide, methacrylamide, maleic acid, acrolein, cyclohexane, ethyl vinyl ether, and methyl vinyl ether. In the present invention, the monomer (C) is preferably acrylamide.

The alkyl portions of the monomers (A) and (B) are preferably short chain length alkyls such as C₁-C₈, more preferably C₁-C₅, still more preferably C₁-C₃, even still more preferably C₁-C₂. When quaternized, the polymers are preferably quaternized with short chain alkyls, i.e., C₁-C₈, more preferably C₁-C₅, still more preferably C₁-C₃, even still more preferably C₁-C₂. The acid addition salts refer to polymers having protonated amino groups. Acid addition salts can be performed through the use of halogen (e.g. chloride), acetic, phosphoric, nitric, citric, or other acids.

When the polymer contains the monomer (C), the molar proportion of the monomer (C) can be from 0% to about 99% based on the total molar proportions of the monomers (A), (B), and (C). The molar proportions of (A) and (B) can independently be from 0% to about 100%. When acrylamide is used as the monomer (C), it will preferably be included at a level of from about 20% to about 99%, more preferably from about 50% to about 99% based on the total molar proportions of the monomers (A), (B), and (C).

Where monomers (A) and (B) are both present, the molar ratio of monomer (A): monomer (B) in the final polymer is preferably from about 95:5 to about 15:85, more preferably from about 80:20 to about 20:80.

Where monomer (A) is not present and monomers (B) and (C) are both present, the molar ratio of monomer (B): monomer (C) in the final polymer is preferably from about 30:70 to about 70:30, more preferably from about 40:60 to about 60:40, still more preferably from about 45:55 to about 55:45.

The crosslinked polymers may also contain a crosslinking agent, which is typically a material containing two or more unsaturated functional groups. The crosslinking agent is reacted with the monomer units of the polymer and is incorporated into the polymer, forming either links or covalent bonds between two or more individual polymer chains or between two or more sections of the same polymer chain. Nonlimiting examples of suitable crosslinking agents include those selected from the group consisting of methylenebisacrylamides, diacrylates, dimethacrylates, di-vinyl aryl (e.g. di-vinyl phenyl ring) compounds, polyalkenyl polyethers of polyhydric alcohols, allyl acrylates, vinyloxyalkylacrylates, and polyfunctional vinylidenes. Specific examples of crosslinking agents useful herein include those selected from the group consisting of methylenebisacrylamide, ethylene glycol, propylene glycol, butylene glycol, di-(meth)acrylate, di-(meth)acrylamide, cyanomethylacrylate, vinyloxyethyleneacrylate, vinyloxyethylenemethacrylate, allyl pentaerythritol, trimethylolpropane, diallylether, allyl sucrose, butadiene, isoprene, 1,4-diethylene benzene, divinyl naphthalene, ethyl vinyl ether, methyl vinyl ether, and allyl acrylate. Other crosslinking agents include formaldehyde and glyoxal. Preferred herein is methylenebisacrylamide.

Widely varying amounts of the crosslinking agents can be employed depending upon the properties desired in the final polymer, e.g. viscosifying effect. The crosslinking agents will typically comprise from about 1 ppm to about 10,000ppm, preferably from about 5ppm to about 750ppm, more preferably from about 25ppm to about 500ppm, even more preferably from about 100ppm to about 500ppm, and preferably from about 250ppm to about 500ppm of the total weight of the polymer on a weight/weight basis.

Exemplary, the crosslinked polymers useful herein include those conforming to the general structure (A)ₘ(B)ₙ(C)ₚ wherein m is zero, (B) is methyl quaternized dimethylaminoethyl methacrylate, the molar ratio of monomers (B):(C) is about 45:55 to about 55:45, and the crosslinking agent is methylenebisacrylamide. An example of such a crosslinking polymer is one that is commercially available as a mineral oil dispersion (which can be include various dispersing aids such as PPG-1 trideceth-6) under the trademark Salcare® SC92 available from Allied Colloids Ltd. This polymer has the CTFA designation, "Polyquaternium 32 (and) Mineral Oil".

Other crosslinked polymers useful herein include those not containing acrylamide or other monomer (C), i.e. p is zero. In these polymers, the monomers (A) and (B) are as described above. An especially preferred group of these polymers is one in which m is also zero. In this instance, the polymer is essentially a homopolymer of dialkylaminoalkyl methacrylate monomer or its quaternary ammonium or acid addition salt. These dialkylaminoalkyl methacrylate copolymers and homopolymers also contain a crosslinking agent as described above.

Preferably, the homopolymer which does not contain acrylamide or other monomer (C) is used in the composition of the present invention. The homopolymers useful herein can be those conforming to the general structure (A)ₘ(B)ₙ(C)ₚ wherein m is zero, (B) is methyl quaternized dimethylaminoethyl methacrylate, p is zero, and the crosslinking agent is methylenebisacrylamide. An example of such a homopolymer is one that is commercially available as a mineral oil dispersion (which can include various dispersing aids such as PPG-1 trideceth-6) under the trademark Salcare® SC95 available from Allied Colloids Ltd. This polymer has the CTFA designation, "Polyquaternium 37 (and) Mineral Oil (and) PPG-1 Trideceth-6". Another example of such a homopolymer is one that is commercially available as an ester dispersion, wherein the ester can be Propylene Glycol Dicaprylate/Dicaprate and the dispersion can include various dispersing aids such as PPG-1 trideceth-6, under the trademark Salcare® SC96 available from Allied Colloids Ltd. This polymer has the CTFA designation, "Polyquaternium 37 (and) Propylene Glycol Dicaprylate/Dicaprate (and) PPG-1 Trideceth-6".

### Hydrophobically modified cellulose ether

The composition of the present invention may comprise a hydrophobically modified cellulose ether as a thickening agent. The hydrophobically modified cellulose ether is preferably included in the composition of the present invention together with the acrylates copolymer and/or the crosslinked polymer.

The hydrophobically modified cellulose ethers useful herein are preferably nonionic polymers. The hydrophobically modified cellulose ethers useful herein comprise a hydrophilic cellulose backbone and a hydrophobic substitution group. The hydrophilic cellulose backbone has a sufficient degree of nonionic substitution to cellulose to be water soluble. Such hydrophilic cellulose backbone is selected from the group consisting of methyl cellulose, hydroxymethyl cellulose, hydroxyethyl cellulose, hydroxyethyl ethylcellulose, hydroxypropyl cellulose, hydroxypropyl methylcellulose, and mixtures thereof. The amount of nonionic substitution is not critical, so long as there is an amount sufficient to assure that the hydrophilic cellulose backbone is water soluble. The hydrophilic cellulose backbone has a molecular weight of about less than 800,000, preferably from about 20,000 to about 700,000, or from about 75 D. P. to about 2500 D. P. Further, where a high viscosity building effect is not desirable, a lower molecular weight cellulose backbone is preferred. One of the preferred hydrophilic cellulose backbone is hydroxyethyl cellulose having a molecular weight of from about 50,000 to about 700,000. Hydroxyethyl cellulose of this molecular weight is known to be one of the most hydrophilic of the materials contemplated. Thus, hydroxyethyl cellulose can be modified to a greater extent than other hydrophilic cellulose backbones.

The hydrophilic cellulose backbone is further substituted with a hydrophobic substitution group via an ether linkage to render the hydrophobically modified cellulose ether to have less than 1% water solubility, preferably less than 0.2% water solubility. The hydrophobic substitution group is selected from a straight or branched chain alkyl group of from about 10 to about 22 carbons; wherein the ratio of the hydrophilic groups in the hydrophilic cellulose backbone to the hydrophobic substitution group being from about 2:1 to about 1000:1, preferably from about 10:1 1 to about 100:1.

Commercially available hydrophobically modified cellulose ethers useful herein include: cetyl hydroxyethylcellulose having tradenames NATROSOL PLUS 330CS and POLYSURF 67, both available from Aqualon Company, Del, USA, having cetyl group substitution of about 0.4% to about 0.65% by weight of the entire polymer.

### Thickening system

The composition of the present invention may comprise a thickening system which comprises at least 2 thickening agents selected from the group consisting of the hydrophobically modified cellulose ether, the acrylates copolymer, and the crosslinked polymer.

In view of providing improved conditioning benefits to the hair while leaving the hair and hands with a clean feeling, and also in view of providing appropriate viscosity and rheology properties, the thickening systems of the composition of the present invention preferably comprise all of these 3 thickening agents.

In view of providing improved conditioning benefits, in the composition of the present invention, the thickening system is preferably a nonionic or cationic system, more preferably a cationic system. The thickening system useful herein has improved compatibility with cationic hair conditioning agents. In the present invention, what is meant by a nonionic system is that the system comprises only nonionic thickening agents, but no cationic thickening agents. In the present invention, what is meant by a cationic system is that the system comprises at least one cationic thickening agent. The cationic system can include nonionic thickening agents. In such preferable nonionic or cationic thickening systems, the hydrophobically modified cellulose ether useful herein is preferably a nonionic thickening agent, and the acrylates copolymer and the crosslinked polymer useful herein are preferably independently a nonionic or cationic thickening agent. More preferably, the hydrophobically modified cellulose ether useful herein is a nonionic thickening agent, and the acrylates copolymer and the crosslinked polymer useful herein are cationic thickening agents. Cationic thickening agents useful herein may provide conditioning benefits.

### FRIZZ CONTROL AGENT

The compositions of the present invention comprise a frizz control agent. The frizz control agent useful herein is believed to provide improved frizz control benefit, hair volume-down benefit, and other conditioning benefits such as reduced static and reduced fly-away benefits. The frizz control agent can be included in the composition of the present invention at a level by weight of, preferably from about 0.1% to about 20%, more preferably from about 0.5% to about 15%, even more preferably from about 1% to about 10%.

The frizz control agent useful herein is selected from the group consisting of (i), (ii) and (iii).
(i) PEG-modified glycerides having the structure: 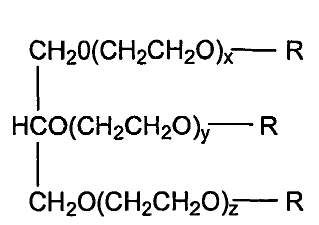 wherein one or more of the R groups is selected from saturated or unsaturated fatty acid moieties derived from animal or vegetable oils such as palmitic acid, lauric acid, oleic acid or linoleic acid wherein the fatty acid moieties have a carbon length chain of from 12 and 22, any other R groups are hydrogen, x, y, z are independently zero or more, the average sum of x+y+z (the degree of ethoxylation) is equal to from about 10 to about 45.
   Preferably, the PEG-modified glycerides have an HLB value of about 20 or less, more preferably about 15 or less, still preferably about 11 or less.
   Preferably the PEG-modified frizz control active has from 2 to 3 fatty acid R groups, more preferred are 3 fatty acid R groups (PEG-modified triglycerides). Preferably, the average sum of x+y+z (the degree of ethoxylation) is equal to from about 20 to 30, more preferred is an average sum of 25. Most preferred are PEG-substituted triglycerides having 3 oleic acid R groups, wherein the average degree of ethoxylation is about 25 (PEG-25 glyceryl trioleate).
   Preferred commercially available PEG-modified triglycerides include Tagat TO ®, Tegosoft GC, Tagat BL 276®, (all three manufactured by Goldschmidt Chemical Corporation) and Crovol A-40, Crovol M-40 (manufactured by Croda Corporation).
(ii) PEG-modified glyceryl fatty acid esters having the structure: 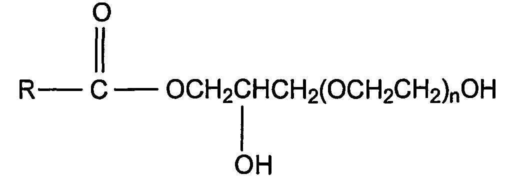 wherein R is an aliphatic group having from 12 to 22 carbon chain length, and n (the degree of ethoxylation) has an average value of from 5 to 40.
   Preferably, the PEG-modified glyceryl fatty acid esters have an HLB value of about 20 or less, more preferably about 15 or less, still preferably about 11 or less.
   Preferably, n has an average value of from about 15 to about 30, more preferred is an average value of from about 20 to about 30, and most preferably has an *average value* of 20. Preferred PEG-modified glyceryl fatty acid esters include PEG-30 glyceryl stearate and PEG-20 glyceryl stearate.
   Preferred commercially available PEG-modified glyceryl fatty acid esters include Tagat S ® and Tagat S 2 ® (manufactured by Goldschmidt Chemical Corporation).
(iii) mixtures thereof.

### AQUEOUS CARRIER

The compositions of the present invention comprise an aqueous carrier. The level and species of the carrier are selected according to the compatibility with other components, and other desired characteristic of the product.

Carriers useful in the present invention include water and water solutions of lower alkyl alcohols. Lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from about 20% to about 99%, preferably from about 40% to about 98%, and more preferably from about 50% to about 98% water.

The pH of the present composition is preferably from about 4 to about 9, more preferably from about 4.5 to about 7.5. Buffers and other pH adjusting agents can be included to achieve the desirable pH.

### ADDITIONAL FRIZZ CONTROL AGENT

The compositions of the present invention may further comprise an additional frizz control agent selected from the group consisting of (i), (ii), and (iii).
(i) polypropylene glycol
   Polypropylene glycol useful herein has a weight average molecular weight of preferably from about 200 g/mol to about 100,000 g/mol, more preferably from about 1,000 g/mol to about 60,000 g/mol. Without intending to be limited by theory, it is believed that the polypropylene glycol herein deposits onto, or is absorbed into hair to act as a moisturizer buffer, and/or provides one or more other desirable hair conditioning benefits. As used herein, the term "polypropylene glycol" includes single-polypropylene glycol-chain segment polymers, and multi-polypropylene glycol-chain segment polymers. The general structure of branched polymers such as the multi-polypropylene glycol-chain segment polymers herein are described, for example, in "Principles of Polymerization," pp. 17-19, G. Odian, (John Wiley & Sons, Inc., 3^{rd} ed., 1991).
   The polypropylene glycol herein are typically polydisperse polymers. The polypropylene glycols useful herein have a polydispersity of from about 1 to about 2.5, preferably from about 1 to about 2, and more preferably from about 1 to about 1.5. As used herein, the term "polydispersity" indicates the degree of the molecular weight distribution of the polymer sample. Specifically, the polydispersity is a ratio, greater than 1, equal to the weight average molecular weight divided by the number average molecular weight. For a further discussion about polydispersity, see "Principles of Polymerization," pp. 20-24, G. Odian, (John Wiley & Sons, Inc., 3^{rd} ed., 1991).
   The polypropylene glycol useful herein may be either water-soluble, water-insoluble, or may have a limited solubility in water, depending upon the degree of polymerization and whether other moieties are attached thereto. The desired solubility of the polypropylene glycol in water will depend in large part upon the form (e.g., leave-on, or rinse-off form) of the hair care composition. The solubility in water of the polypropylene glycol herein may be chosen by the artisan according to a variety of factors. Accordingly, for a leave-on hair care composition, it is preferred that the polypropylene glycol herein be a water-soluble polypropylene glycol. Solubility information is readily available from polypropylene glycol suppliers, such as Sanyo Kasei (Osaka, Japan). However, the present invention may also take the form of a rinse-off hair care composition. Without intending to be limited by theory, it is believed that in such a composition, a water-soluble polypropylene glycol may be too easily washed away before it effectively deposits on hair and provides the desired benefit(s). For such a composition, a less soluble, or even a water-insoluble polypropylene glycol is therefore preferred. Accordingly, for a rinse-off hair care composition, it is preferred that the polypropylene glycol herein has a solubility in water at 25 °C of less than about 1 g/100 g water, more preferably a solubility in water of less than about 0.5 g/100 g water, and even more preferably a solubility in water of less than about 0.1 g/100 g water.
   Preferably the polypropylene glycol is selected from the group consisting of a single-polypropylene glycol-chain segment polymer, a multi-polypropylene glycol-chain segment polymer, and mixtures thereof, more preferably selected from the group consisting of a single-polypropylene glycol-chain segment polymer of Formula I, below, a multi-polypropylene glycol-chain segment polymer of Formula II, below, and mixtures thereof.
   Accordingly, a highly preferred single-polypropylene glycol-chain segment polymer has the formula:

   HO-(C₃H₆O)ₐH (III),

   wherein a is a value from about 4 to about 400, preferably from about 20 to about 100, and more preferably from about 20 to about 40.
   The single-polypropylene glycol-chain segment polymer useful herein is typically inexpensive, and is readily available from, for example, Sanyo Kasei (Osaka, Japan), Dow Chemicals (Midland, Michigan, USA), Calgon Chemical, Inc. (Skokie, Illinois, USA), Arco Chemical Co. (Newton Square Pennsylvania, USA), Witco Chemicals Corp. (Greenwich, Connecticut, USA), and PPG Specialty Chemicals (Gurnee, Illinois, USA).
   A highly preferred multi-polypropylene glycol-chain segment polymer has the formula: 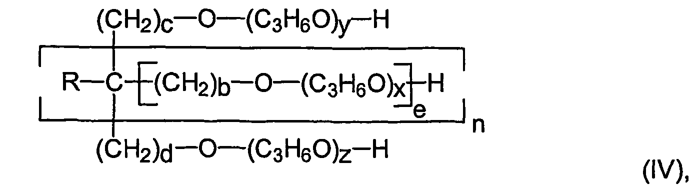 wherein n is a value from about 0 to about 10, preferably from about 0 to about 7, and more preferably from about 1 to about 4. In Formula IV, each R" is independently selected from the group consisting of H, and C₁-C₃₀ alkyl, and preferably each R" is independently selected from the group consisting of H, and C₁-C₄ alkyl. In Formula IV, each b is independently a value from about 0 to about 2, preferably from about 0 to about 1, and more preferably b = 0. Similarly, c and d are independently a value from about 0 to about 2, preferably from about 0 to about 1. However, the total of b + c + d is at least about 2, preferably the total of b + c + d is from about 2 to about 3. Each e is independently a value of 0 or 1, if n is from about 1 to about 4, then e is preferably equal to 1. Also in Formula IV, x, y, and z is independently a value of from about 1 to about 120, preferably from about 7 to about 100, and more preferably from about 7 to about 100, where x + y + z is greater than about 20.
   Examples of the multi-polypropylene glycol-chain segment polymer of Formula IV which is especially useful herein includes polyoxypropylene glyceryl ether (n = 1, R' = H, b = 0, c and d = 1, e = 1, and x, y, and z independently indicate the degree of polymerization of their respective polypropylene glycol-chain segments; available as New Pol GP-4000, from Sanyo Kasei, Osaka, Japan), polypropylene trimethylol propane (n = 1, R' = C₂H₅, b = 1, c and d = 1, e = 1, and x, y, and z independently indicate the degree of polymerization of their respective polypropylene glycol-chain segments), polyoxypropylene sorbitol (n = 4, each R' = H, b = 0, c and d = 1, each e = 1, and y, z, and each x independently indicate the degree of polymerization of their respective polypropylene glycol-chain segments; available as New Pol SP-4000, from Sanyo Kasei, Osaka, Japan), and PPG-10 butanediol (n = 0, c and d = 2, and y + z = 10; available as Probutyl DB-10, from Croda, Inc., of Parsippany, New Jersey, U.S.A.).
   In a preferred embodiment, one or more of the propylene repeating groups in the polypropylene glycol is an isopropyl oxide repeating group. More preferably one or more of the propylene oxide repeating groups of the polypropylene glycol of Formula III and/or the polypropylene glycol of Formula IV is an isopropyl oxide repeating group. Even more preferably, substantially all of the propylene oxide repeating groups of the polypropylene glycol of Formula III and/or the polypropylene glycol of Formula IV are isopropyl oxide repeating groups. Accordingly, a highly preferred single-polypropylene glycol-chain segment polymer has the formula: 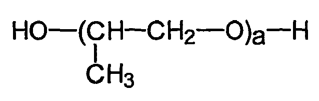 wherein a is defined as described above for Formula III. Similarly, a highly preferred multi-polypropylene glycol-chain segment polymer has the formula: 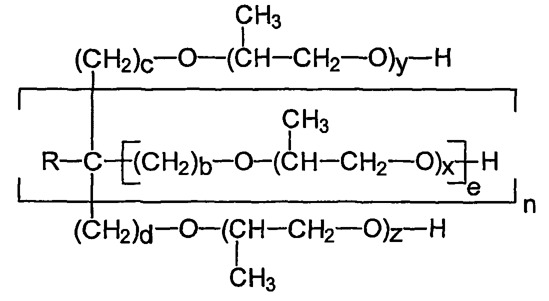 wherein n, R", b, c, d, e, x, y, and z are defined as above, for Formula IV. It is recognized that the isopropyl oxide repeating groups may also correspond either alone, or in combination with the above depicted, to: 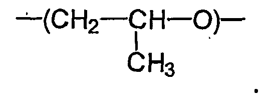
   The polypropylene glycol useful herein is readily available from, for example, Sanyo Kasei (Osaka, Japan) as New pol PP-2000, New pol PP-4000, New pol GP-4000, and New pol SP-4000, from Dow Chemicals (Midland, Michigan, USA), from Calgon Chemical, Inc. (Skokie, Illinois, USA), from Arco Chemical Co. (Newton Square Pennsylvania, USA), from Witco Chemicals Corp. (Greenwich, Connecticut, USA), and from PPG Specialty Chemicals (Gurnee, Illinois, USA).
(ii) Pentaerythritol ester oils useful herein are those having the formula: 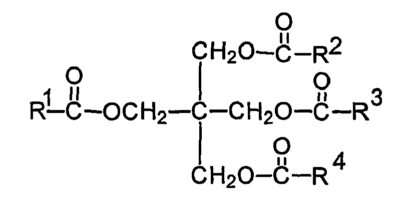 wherein R¹, R², R³, and R⁴, independently, are branched, straight, saturated, or unsaturated alkyl, aryl, and alkylaryl groups having from 1 to about 30 carbons. Preferably, R¹, R², R³, and R⁴, independently, are branched, straight, saturated, or unsaturated alkyl groups having from about 8 to about 22 carbons. More preferably, R¹, R², R³ and R⁴ are defined so that the molecular weight of the compound is from about 800 to about 1200.
   Particularly useful pentaerythritol ester oils herein include pentaerythritol tetraisostearate, pentaerythritol tetraoleate, and mixtures thereof. Such compounds are available from Kokyu Alcohol with tradenames KAKPTI, KAKTTI.
(iii) mixtures thereof.

The additional frizz control agent can be included in the composition of the present invention at a level by weight of, preferably from about 0.1 % to about 20%, more preferably from about 0.5% to about 15%, even more preferably from about 1% to about 10%.

### SILICONE COMPOUND

Preferably, the compositions of the present invention contain a silicone compound. The silicone compounds useful herein include volatile soluble or insoluble, or nonvolatile soluble or insoluble silicone conditioning agents. By soluble what is meant is that the silicone compound is miscible with the carrier of the composition so as to form part of the same phase. By insoluble what is meant is that the silicone forms a separate, discontinuous phase from the carrier, such as in the form of an emulsion or a suspension of droplets of the silicone. The silicone compounds herein may be made by any suitable method known in the art, including emulsion polymerization. The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

The silicone compounds herein are preferably used at levels by weight of the composition of from about 0.1 % to about 40%, more preferably from about 0.1 % to about 10%, still more preferably from about 0.1 % to about 5%.

A nonvolatile dispersed silicone that can be especially useful is a silicone gum. The term "silicone gum", as used herein, means a polyorganosiloxane material having a viscosity at 25°C of greater than or equal to 1,000,000 mPa·s. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer *et al.,* issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof.

Also useful are silicone resins, which are highly crosslinked polymeric siloxane systems. The crosslinking is introduced through the incorporation of trifunctional and tetra-functional silanes with mono-functional or di-functional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units, and hence, a sufficient level of crosslinking, such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Preferably, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinylchlorosilanes, and tetrachlorosilane, with the methyl substituted silanes being most commonly utilized. Preferred resins are offered by General Electric as GE SS4230 and SS4267. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Without being bound by theory, it is believed that the silicone resins can enhance deposition of other silicone compounds on the hair and can enhance the glossiness of hair with high refractive index volumes.

Other useful silicone resins are silicone resin powders such as the material given the CTFA designation polymethylsilsequioxane, which is commercially available as Tospearl™ from Toshiba Silicones.

The method of manufacturing these silicone compounds, can be found in Encyclopedia of Polymer Science and Engineering, Volume 15, Second Edition, pp. 204-308, John Wiley & Sons, Inc., 1989.

Silicone materials and silicone resins in particular, can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SlO_{0.5}; D denotes the difunctional unit (CH₃)₂SiO; T denotes the trifunctional unit (CH₃)SiO_{1.5}; and Q denotes the quadri- or tetra-functional unit Si02. Primes of the unit symbols, e.g., M', D', T', and Q' denote substituents other than methyl, and must be specifically defined for each occurrence. Typical alternate substituents include groups such as vinyl, phenyl, amino, hydroxyl, etc. The molar ratios of the various units, either in terms of subscripts to the symbols indicating the total number of each type of unit in the silicone, or an average thereof, or as specifically indicated ratios in combination with molecular weight, complete the description of the silicone material under the MDTQ system. Higher relative molar amounts of T, Q, T' and/or Q' to D, D', M and/or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 10,000.

The silicone compounds herein also include polyalkyl or polyaryl siloxanes with the following structure (I) 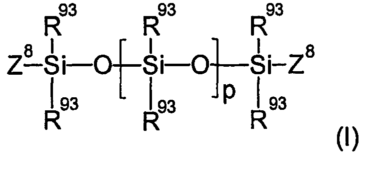 wherein R⁹³ is alkyl or aryl, and x is an integer from about 7 to about 8,000. Z⁸ represents groups which block the ends of the silicone chains. The alkyl or aryl groups substituted on the siloxane chain (R⁹³) or at the ends of the siloxane chains Z⁸ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable Z⁸ groups include hydroxy, methyl, methoxy, ethoxy, propoxy, and aryloxy. The two R⁹³ groups on the silicon atom may represent the same group or different groups. Preferably, the two R⁹³ groups represent the same group. Suitable R⁹³ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from the General Electric Company in their Viscasil® and SF 96 series, and from Dow Corning in their Dow Corning 200 series.

Polyalkylaryl siloxane fluids can also be used and include, for example, polymethylphenylsiloxanes. These siloxanes are available, for example, from the General Electric Company as SF 1075 methyl phenyl fluid or from Dow Corning as 556 Cosmetic Grade Fluid.

Especially preferred, for enhancing the shine characteristics of hair, are highly arylated silicone compounds, such as highly phenylated polyethyl silicone having refractive index of about 1.46 or higher, especially about 1.52 or higher. When these high refractive index silicone compounds are used, they should be mixed with a spreading agent, such as a surfactant or a silicone resin, as described below to decrease the surface tension and enhance the film forming ability of the material.

Other silicone compounds include amino substituted materials. Suitable alkylamino substituted silicone compounds include those represented by the following structure (II) 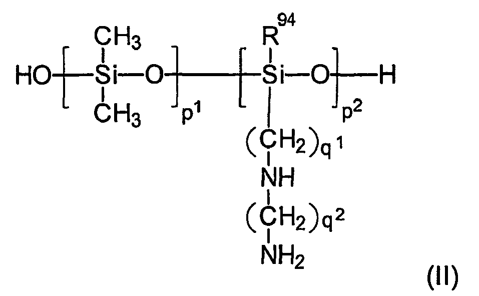 wherein R⁹⁴ is H, CH₃ or OH, p¹, p², q¹ and q² are integers which depend on the molecular weight, the average molecular weight being approximately between 5,000 and 10,000. This polymer is also known as "amodimethicone".

Suitable amino substituted silicone fluids include those represented by the formula (III)

(R⁹⁷)ₐG₃₋ₐ―Si―(OSiG₂)ₚ₃―(OSiG_{b}(R⁹⁷)_{2-b})ₚ₄―O―SiG₃₋ₐ(R⁹⁷)ₐ (III)

in which G is chosen from the group consisting of hydrogen, phenyl, OH, C₁-C₈ alkyl and preferably methyl; a denotes 0 or an integer from 1 to 3, and preferably equals 0; b denotes 0 or 1 and preferably equals 1; the sum p³+p⁴ is a number from 1 to 2,000 and preferably from 50 to 150, p³ being able to denote a number from 0 to 1,999 and preferably from 49 to 149 and p⁴ being able to denote an integer from 1 to 2,000 and preferably from 1 to 10; R⁹⁷ is a monovalent radical of formula C_{q3}H_{2q3}L in which q³ is an integer from 2 to 8 and L is chosen from the groups

―N(R⁹⁶)CH₂―CH₂―N(R⁹⁶)₂

―N(R⁹⁶)₂

―N(R⁹⁶)₃X'

―N(R⁹⁶)CH₂―CH₂―NR⁹⁶H₂X'

in which R⁹⁶ is chosen from the group consisting of hydrogen, phenyl, benzyl, a saturated hydrocarbon radical, preferably an alkyl radical containing from 1 to 20 carbon atoms, and X' denotes a halide ion.

An especially preferred amino substituted silicone corresponding to formula (II) is the polymer known as "trimethylsilylamodimethicone" wherein R⁹⁴ is CH₃.

Other amino substituted silicone polymers which can be used are represented by the formula (V): 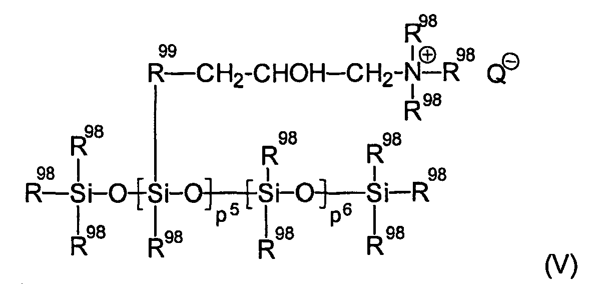 where R⁹⁸ denotes a monovalent hydrocarbon radical having from 1 to 18 carbon atoms, preferably an alkyl or alkenyl radical such as methyl; R⁹⁹ denotes a hydrocarbon radical, preferably a C₁-C₁₈ alkylene radical or a C₁-C₁₈, and more preferably C₁-C₈, alkyleneoxy radical; Q⁻ is a halide ion, preferably chloride; p⁵ denotes an average statistical value from 2 to 20, preferably from 2 to 8; p⁶ denotes an average statistical value from 20 to 200, and preferably from 20 to 50. A preferred polymer of this class is available from Union Carbide under the name "UCAR SILICONE ALE 56."

References disclosing suitable nonvolatile dispersed silicone compounds include U.S. Patent No. 2,826,551, to Geen; U.S. Patent No. 3,964,500, to Drakoff, issued June 22, 1976; U.S. Patent No. 4,364,837, to Pader;.and British Patent No. 849,433, to Woolston. "Silicon Compounds" distributed by Petrarch Systems, Inc., 1984, provides an extensive, though not exclusive, listing of suitable silicone compounds.

The silicone compounds for use herein will preferably have a viscosity of from about 1,000 to about 2,000,000 mPa•s at 25°C, more preferably from about 10,000 to about 1,800,000, and even more preferably from about 100,000 to about 1,500,000. The viscosity can be measured by means of a glass capillary viscometer as set forth in Dow Corning Corporate Test Method CTM0004, July 20, 1970. Silicone compound of high molecular weight may be made by emulsion polymerization. Suitable silicone fluids include polyalkyl siloxanes, polyaryl siloxanes, polyalkylaryl siloxanes, polyether siloxane copolymers, and mixtures thereof. Other nonvolatile silicone compounds having hair conditioning properties can also be used.

Particularly suitable silicone compounds herein are non-volatile silicone oils having a molecular weight of from about 200,000 to about 600,000 such as Dimethicone, and Dimethiconol. These silicone compounds can be incorporated in the composition as silicone oils solutions; the silicone oils being volatile or non-volatile.

Commercially available silicone compounds which are useful herein include Dimethicone with tradename DC200 available from Dow Corning Corporation, Dimethicone gum solutions with tradenames SE 30, SE 33, SE 54 and SE 76 available from General Electric, Dimethiconol with tradenames DCQ2-1403 and DCQ2-1401 available from Dow Corning Corporation, Mixture of Dimethicone and Dimethiconol with tradename DC1403 available from Dow Corning Corporation, and emulsion polymerized Dimethiconol available from Toshiba Silicone as described in GB application 2,303,857.

### HUMECTANT

Preferably, the compositions of the present invention contain a humectant. The humectants herein are selected from the group consisting of polyhydric alcohols, water soluble alkoxylated nonionic polymers, and mixtures thereof. The humectants herein are preferably used at levels by weight of the composition of from about 0.1 % to about 20%, more preferably from about 0.5% to about 5%. Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sultate, sodium hyaluronate, sodium adenosin phosphate, sodium lactate, pyrrolidone carbonate, glucosamine, cyclodextrin, and mixtures thereof.

Water soluble alkoxylated nonionic polymers useful herein include polyethylene glycols and polypropylene glycols having a molecular weight of up to about 1000 such as those with CTFA names PEG-200, PEG-400, PEG-600, PEG-1000, and mixtures thereof.

Commercially available humectants herein include: glycerin with tradenames STAR and SUPEROL available from The Procter & Gamble Company, CRODEROL GA7000 available from Croda Universal Ltd., PRECERIN series available from Unichema, and a same tradename as the chemical name available from NOF; propylene glycol with tradename LEXOL PG-865/855 available from Inolex, 1,2-PROPYLENE GLYCOL USP available from BASF; sorbitol with tradenames LIPONIC series available from Lipo, SORBO, ALEX, A-625, and A-641 available from ICI, and UNISWEET 70, UNISWEET CONC available from UPI; dipropylene glycol with the same tradename available from BASF; diglycerin with tradename DIGLYCEROL available from Solvay GmbH; xylitol with the same tradename available from Kyowa and Eizai; maltitol with tradename MALBIT available from Hayashibara, sodium chondroitin sulfate with the same tradename available from Freeman and Bioiberica, and with tradename ATOMERGIC SODIUM CHONDROITIN SULFATE available from Atomergic Chemetals; sodium hyaluronate with tradenames ACTIMOIST available from Active Organics, AVIAN SODIUM HYALURONATE series available from Intergen, HYALURONIC ACID Na available from Ichimaru Pharcos; sodium adenosin phophate with the same tradename available from Asahikasei, Kyowa, and Daiichi Seiyaku; sodium lactate with the same tradename available from Merck, Wako, and Showa Kako, cyclodextrin with tradenames CAVITRON available from American Maize, RHODOCAP series available from Rhone-Poulenc, and DEXPEARL available from Tomen; and polyethylene glycols with the tradename CARBOWAX series available from Union Carbide.

### AMPHOTERIC CONDITIONING POLYMER

The compositions of the present invention may further contain an amphoteric conditioning polymer. The amphoteric conditioning polymers herein are those compatible with the thickening system and which provide conditioning benefit to the hair. Although some of the amphoteric conditioning polymers herein may have some hair holding or hair fixative properties, such hair holding or hair fixative properties are not a requirement for the amphoteric conditioning polymers herein. The amphoteric conditioning polymers useful herein are those including at least one cationic monomer and at least one anionic monomer; the cationic monomer being quaternary ammonium, preferably dialkyl diallyl ammonium chloride or carboxylamidoalkyl trialkyl ammonium chloride; and the anionic monomer being carboxylic acid. The amphoteric conditioning polymers herein may include nonionic monomers such as acrylamine, methacrylate, or ethacrylate. Further, the amphoteric conditioning polymers useful herein do not contain betanized monomers.

The composition of the present invention preferably comprises the amphoteric conditioning polymer at a level by weight of from about 0.01% to about 10%, more preferably from about 0.1 % to about 5%.

Useful herein are polymers with the CTFA name Polyquaternium 22, Polyquaternium 39, and Polyquaternium 47. Such polymers are, for example, copolymers consisting of dimethyldiallyl ammonium chloride and acrylic acid, terpolymers consisting of dimethyldiallyl ammonium chloride and acrylamide, and terpolymers consisting of acrylic acid methacrylamidopropyl trimethylammonium chloride and methyl acrylate such as those of the following formula wherein the ratio of n⁶:n⁷:n⁸ is 45:45:10:

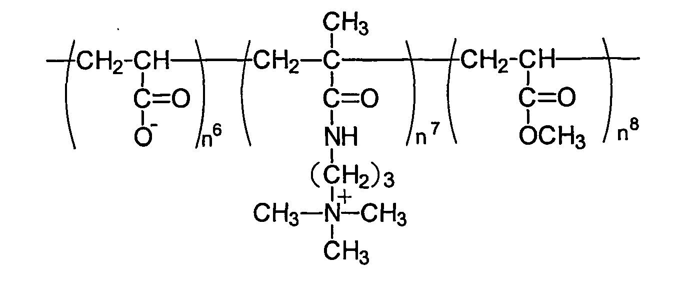

Highly preferred commercially available amphoteric conditioning polymers herein include Polyquaternium 22 with tradenames MERQUAT 280, MERQUAT 295, Polyquaternium 39 with tradenames MERQUAT PLUS 3330, MERQUAT PLUS 3331, and Polyquaternium 47 with tradenames MERQUAT 2001, MERQUAT 2001 N, all available from Calgon Corporation.

Also useful herein are polymers resulting from the copolymerisation of a vinyl monomer carrying at least one carboxyl group, such as acrylic acid, methacrylic acid, maleic acid, itaconic acid, fumaric acid, crotonic acid, or alphachloroacrylic acid, and a basic monomer which is a substituted vinyl compound containing at least one basic nitrogen atom, such as dialkylaminoalkyl methacrylates and acrylates and dialkylaminoalkylmethacrylamides and acrylamides.

Also useful herein are polymers containing units derived from:
i) at least one monomer chosen from amongst acrylamides or methacrylamides substituted on the nitrogen by an alkyl radical,
ii) at least one acid comonomer containing one or more reactive carboxyl groups, and
iii) at least one basic comonomer, such as esters, with primary, secondary and tertiary amine substituents and quaternary ammonium substituents, of acrylic and methacrylic acids, and the product resulting from the quaternisation of dimethylaminoethyl methacrylate with dimethyl or diethyl sulfate.

The N-substituted acrylamides or methacrylamides which are most particularly preferred are the groups in which the alkyl radicals contain from 2 to 12 carbon atoms, especially N-ethylacrylamide, N-tert.-butylacrylamide, N-tert.-octylacrylamide, N-octylacrylamide, N-decylacrylamide and N-dodecylacrylamide and also the corresponding methacrylamides. The acid comonomers are chosen more particularly from amongst acrylic, methacrylic, crotonic, itaconic, maleic and fumaric acids and also the alkyl monoesters of maleic acid or fumaric acid in which alkyl has 1 to 4 carbon atoms.

The preferred basic comonomers are aminoethyl, butylaminoethyl, N,N'-dimethylaminoethyl and N-tert.-butylaminoethyl methacrylates. Commercially available amphoteric conditioning polymers herein include octylacrylamine/acrylates/butylaminoethyl methacrylate copolymers with the tradenames AMPHOMER, AMPHOMER SH701, AMPHOMER 28-4910, AMPHOMER LV71, and AMPHOMER LV47 supplied by National Starch & Chemical.

### CATIONIC CONDITIONING AGENT

The compositions of the present invention may further contain a cationic conditioning agent selected from the group consisting of cationic surfactants, cationic polymers, and mixtures thereof. Cationic conditioning agents are selected according to the compatibility with other components, and the desired characteristic of the product. Preferred herein is a cationic surfactant. The cationic conditioning agents herein are preferably used at levels by weight of the composition of from about 0.01 % to about 10%.

### Cationic Surfactant

Among the cationic surfactants useful herein are those corresponding to the general formula (I): 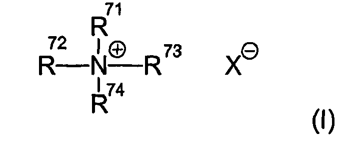 wherein at least one of R⁷¹, R⁷², R⁷³ and R⁷⁴ is selected from an aliphatic group of from 8 to 30 carbon atoms or an aromatic, alkoxy, polyoxyalkylene, alkylamido, hydroxyalkyl, aryl or alkylaryl group having up to about 22 carbon atoms, the remainder of R⁷¹, R⁷², R⁷³ and R⁷⁴ are independently selected from an aliphatic group of from 1 to about 22 carbon atoms or an aromatic, alkoxy, polyoxyalkylene, alkylamido, hydroxyalkyl, aryl or alkylaryl group having up to about 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether linkages, and other groups such as amino groups. The longer chain aliphatic groups, e.g., those of about 12 carbons, or higher, can be saturated or unsaturated. Preferred is when R⁷¹, R⁷², R⁷³ and R⁷⁴ are independently selected from C₁ to about C₂₂alkyl. Nonlimiting examples of cationic surfactants useful in the present invention include the materials having the following CTFA designations: quaternium-8, quaternium-14, quaternium-18, quatemium-18 methosulfate, quaternium-24, and mixtures thereof.

Among the cationic surfactants of general formula (I), preferred are those containing in the molecule at least one alkyl chain having at least 16 carbons. Nonlimiting examples of such preferred cationic surfactants include: behenyl trimethyl ammonium chloride available, for example, with tradename INCROQUAT TMC-80 from Croda and ECONOL TM22 from Sanyo Kasei; cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methy) pyridinium chloride.

Also preferred are hydrophilically substituted cationic surfactants in which at least one of the substituents contain one or more aromatic, ether, ester, amido, or amino moieties present as substituents or as linkages in the radical chain, wherein at least one of the R⁷¹-R⁷⁴ radicals contain one or more hydrophilic moieties selected from alkoxy (preferably C₁-C₃ alkoxy), polyoxyalkylene (preferably C₁-C₃ polyoxyalkylene), alkylamido, hydroxyalkyl, alkylester, and combinations thereof. Preferably, the hydrophilically substituted cationic conditioning surfactant contains from 2 to about 10 nonionic hydrophile moieties located within the above stated ranges. Preferred hydrophilically substituted cationic surfactants include those of the formula (II) through (VIII) below: 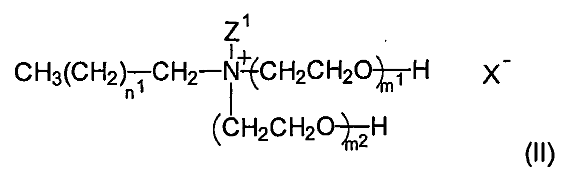 wherein n¹ is from 8 to about 28, m¹+m² is from 2 to about 40, Z¹ is a short chain alkyl, preferably a C₁-C₃ alkyl, more preferably methyl, or (CH₂CH₂O)ₘ₃H wherein m¹+m²+m³ is up to 60, and X is a salt forming anion as defined above; 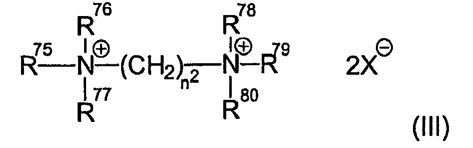 wherein n² is 1 to 5, one or more of R⁷⁵, R⁷⁶, and R⁷⁷ are independently an C₁-C₃₀ alkyl, the remainder are CH₂CH₂OH, one or two of R⁷⁸, R⁷⁹, and R⁸⁰ are independently an C₁-C₃₀ alkyl, and remainder are CH₂CH₂OH, and X is a salt forming anion as mentioned above; 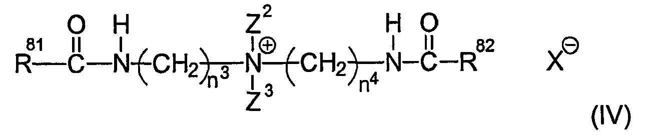 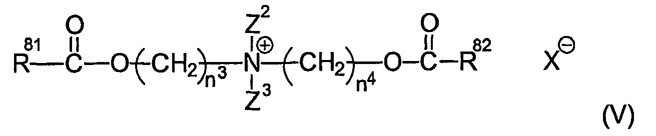 wherein, independently for formulae (IV) and (V), Z² is an alkyl, preferably C₁-C₃ alkyl, more preferably methyl, and Z³ is a short chain hydroxyalkyl, preferably hydroxymethyl or hydroxyethyl, n³ and n⁴ independently are integers from 2 to 4, inclusive, preferably from 2 to 3, inclusive, more preferably 2, R⁸¹ and R⁸², independently, are substituted or unsubstituted hydrocarbyls, C₁₂-C₂₀ alkyl or alkenyl, and X is a salt forming anion as defined above; 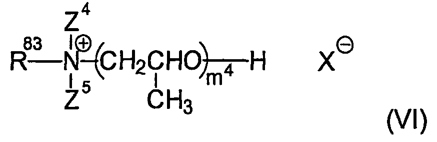 wherein R⁸³ is a hydrocarbyl, preferably a C₁-C₃ alkyl, more preferably methyl, Z⁴ and Z⁵ are, independently, short chain hydrocarbyls, preferably C₂-C₄ alkyl or alkenyl, more preferably ethyl, m⁴ is from 2 to about 40, preferably from about 7 to about 30, and X is a salt forming anion as defined above; 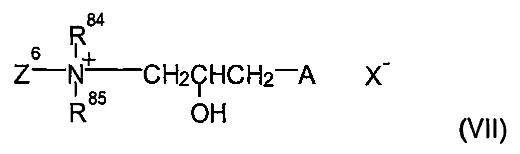 wherein R⁸⁴ and R⁸⁵, independently, are C₁-C₃ alkyl, preferably methyl, Z⁶ is a C₁₂-C₂₂ hydrocarbyl, alkyl carboxy or alkylamido, and A is a protein, preferably a collagen, keratin, milk protein, silk, soy protein, wheat protein, or hydrolyzed forms thereof; and X is a salt forming anion as defined above; 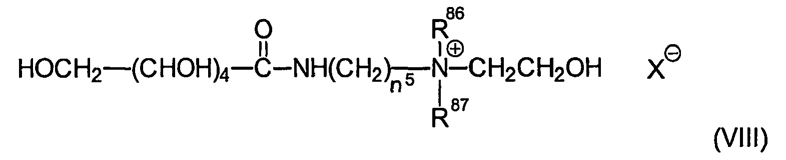 wherein n⁵ is 2 or 3, R⁸⁶ and R⁸⁷, independently are C₁-C₃ hydrocarbyls preferably methyl, and X is a salt forming anion as defined above. Nonlimiting examples of hydrophilically substituted cationic surfactants useful in the present invention include the materials having the following CTFA designations: quaternium-16, quaternium-26, quaternium-27, quaternium-30, quaternium-33, quatemium-43, quaternium-52, quaternium-53, quaternium-56, quaternium-60, quaternium-61, quaternium-62, quaternium-70, quaternium-71, quaternium-72, quaternium-75, quaternium-76 hydrolyzed collagen, quaternium-77, quaternium-78, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, and quaternium-79 hydrolyzed wheat protein, quaternium-80, quaternium-81, quaternium-82, quaternium-83, quaternium-84, and mixtures thereof.

Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commercially available under the following tradenames; VARISOFT 110, VARISOFT 222, VARIQUAT K1215 and VARIQUAT 638 from Witco Chemical, MACKPRO KLP, MACKPRO WLW, MACKPRO MLP, MACKPRO NSP, MACKPRO NLW, MACKPRO WWP, MACKPRO NLP, MACKPRO SLP from McIntyre, ETHOQUAD 18/25, ETHOQUAD O/12PG, ETHOQUAD C/25, ETHOQUAD S/25, and ETHODUOQUAD from Akzo, DEHYQUAT SP from Henkel, and ATLAS G265 from ICI Americas.

Amines are suitable as cationic surfactants. Primary, secondary, and tertiary fatty amines are useful. Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidylbehenylamine. Useful amines in the present invention are disclosed in U.S. Patent 4,275,055, Nachtigal, et al.

These amines can also be used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid. The amines herein are preferably partially neutralized with any of the acids at a molar ratio of the amine to the acid of from about 1 : 0.3 to about 1 : 2, more preferably from about 1 : 0.4 to about 1:1.

### Cationic Polymers

Cationic polymers are also useful herein. As used herein, the term "polymer" shall include materials whether made by polymerization of one type of monomer or made by two *(i.e.,* copolymers) or more types of monomers. The cationic polymers useful herein may include the polymers disclosed above under the title "ACRYLATES COPOLYMER" and "CROSSLINKED POLYMER", however, the cationic polymers useful herein are preferably different from such polymers when such polymers are cationic.

Preferably, the cationic polymer is a water soluble cationic polymer. By "water soluble" cationic polymer, what is meant is a polymer which is sufficiently soluble in water to form a substantially clear solution to the naked eye at a concentration of 0.1% in water (distilled or equivalent) at 25°C. The preferred polymer will be sufficiently soluble to form a substantially clear solution at 0.5% concentration, more preferably at 1.0% concentration.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, and mixtures thereof.

Any anionic counterions can be utilized for the cationic polymers so long as the water solubility criteria is met. Suitable counterions include halides (e.g., Cl, Br, I, or F, preferably Cl, Br, or I), sulfate, and methylsulfate. Others can also be used, as this list is not exclusive. The cationic nitrogen-containing moiety will be present generally as a substituent, on a fraction of the total monomer units of the cationic hair conditioning polymers. Thus, the cationic polymer can comprise copolymers, terpolymers, etc. of quaternary ammonium or cationic amine-substituted monomer units and other non-cationic units referred to herein as spacer monomer units. Such polymers are known in the art, and a variety can be found in the CTFA Cosmetic Ingredient Dictionary, 3rd edition, edited by Estrin, Crosley, and Haynes, (The Cosmetic, Toiletry, and Fragrance Association, Inc., Washington, D.C., 1982).

The cationic amines can be primary, secondary, or tertiary amines, depending upon the particular species and the pH of the composition. In general, secondary and tertiary amines, especially tertiary amines, are preferred.

Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R⁸⁸X wherein R⁸⁸ is a short chain alkyl, preferably a C₁ - C₇ alkyl, more preferably a C₁ - C₃ alkyl, and X is a salt forming anion as defined above.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl portions of these monomers are preferably lower alkyls such as the C₁ - C₃ alkyls, more preferably C₁ and C₂ alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C₁ - C₇ hydrocarbyls, more preferably C₁ - C₃, alkyls.

The cationic polymers hereof can comprise mixtures of monomer units derived from amine- and/or quaternary ammonium-substituted monomer and/or compatible spacer monomers.

Suitable cationic hair conditioning polymers include, for example: copolymers of 1-vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e.g., LUVIQUAT FC 370); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; and mineral acid salts of amino-alkyl esters of homo- and co-polymers of unsaturated carboxylic acids having from 3 to 5 carbon atoms, as described in U.S. Patent 4,009,256.

Other cationic polymers that can be used include polysaccharide polymers, such as cationic cellulose derivatives and cationic starch derivatives.

Cationic polysaccharide polymer materials suitable for use herein include those of the formula: 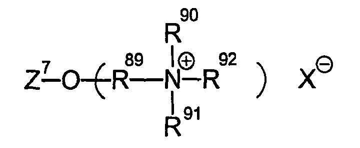 wherein: Z⁷ is an anhydroglucose residual group, such as a starch or cellulose anhydroglucose residual, R⁸⁹ is an alkylene oxyalkylene, polyoxyalkylene, or hydroxyalkylene group, or combination thereof, R⁹⁰, R⁹¹, and R⁹² independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R⁹⁰, R⁹¹ and R⁹²) preferably being about 20 or less, and X is as previously described.

Cationic cellulose is available from Amerchol Corp. (Edison, NJ, USA) in their Polymer JR® and LR® series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200®.

Other cationic polymers that can be used include cationic guar gum derivatives, such as guar hydroxypropyltrimonium chloride commercially available from Celanese Corp. in their Jaguar R series. Other materials include quaternary nitrogen-containing cellulose ethers as described in U.S. Patent 3,962,418, and copolymers of etherified cellulose and starch as described in U.S. Patent 3,958,581.

Particularly useful cationic polymers herein include Polyquaternium-7, Polyquaternium-10, Polyquaternium-24, and mixtures thereof.

### ADDITIONAL VISCOSITY MODIFIER

The compositions of the present invention may further contain an additional viscosity modifier. The additional viscosity modifiers herein are water soluble or water miscible polymers, have the ability to increase the viscosity of the composition, and are compatible with the thickening system of the present invention.

The additional viscosity modifiers useful herein include anionic polymers and nonionic polymers. Preferred additional viscosity modifiers useful herein are nonionic polymers. Anionic polymers can be used when the thickening system of the present invention is nonionic. The additional viscosity modifiers useful herein may include the polymers disclosed above under the title, "Acrylic acid/alkyl acrylate copolymer", "Acrylates copolymer", "Crosslinked polymer", and "Hydrophobically modified cellulose ether" however, the additional viscosity modifiers useful herein are preferably different from such polymers.

The additional viscosity modifier is selected so that the composition of the present composition has a suitable viscosity, preferably from about 1,000 cps to about 100,000 cps, more preferably from about 2,000 cps to about 50,000cps. If such a viscosity is achieved without the additional viscosity modifier, the additional viscosity modifier may not be necessary. The viscosity herein can be suitably measured by Brookfield RVT at 20rpm at 20°C using either spindle #4, 5, 6 or 7 depending on the viscosity and the characteristic of the composition.

The additional viscosity modifiers herein are preferably used at levels by weight of the composition of from about 0.001% to about 5%, more preferably from about 0.05% to about 3%.

Useful herein are carboxylic acid/carboxylate copolymers such as hydrophobically-modified cross-linked coplymers of carboxylic acid and alkyl carboxylate, and have an amphiphilic property. These carboxylic acid/carboxylate copolymers are obtained by copolymerizing 1) a carboxylic acid monomer such as acrylic acid, methacrylic acid, maleic acid, maleic anhydride, itaconic acid, fumaric acid, crotonic acid, or α-chloroacrylic acid, 2) a carboxylic ester having an alkyl chain of from 1 to about 30 carbons, and preferably 3) a crosslinking agent of the following formula: 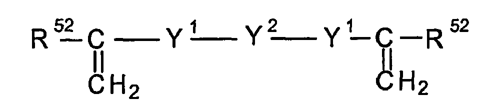 wherein R⁵² is a hydrogen or an alkyl group having from about 1 to about 30 carbons; Y¹, indepedently, is oxygen, CH₂O, COO, OCO, 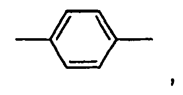 or 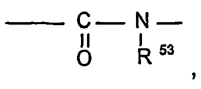 wherein R⁵³ is a hydrogen or an alkyl group having from about 1 to about 30 carbons; and Y² is selected from (CH₂)ₘ", (CH₂CH₂O)ₘ'', or (CH₂CH₂CH₂O)ₘ" wherein m" is an integer of from 1 to about 30.

Neutralizing agents may be included to neutralize the carboxylic acid/carboxylate copolymers herein. Nonlimiting examples of such neutralizing agents include sodium hydroxide, potassium hydroxide, ammonium hydroxide, monethanolamine, diethanolamine, triethanolamine, diisopropanolamine, aminomethylpropanol, tromethamine, tetrahydroxypropyl ethylenediamine, and mixtures thereof.

Another additional viscosity modifier useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Polyethylene glycols having a molecular weight of more than about 1000 are useful herein. Useful are those having the following general formula: 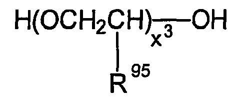 wherein R⁹⁵ is selected from the group consisting of H, methyl, and mixtures thereof. In the above structure, x3 has an average value of from about 1500 to about 25,000, preferably from about 2500 to about 20,000, and more preferably from about 3500 to about 15,000. When R⁹⁵ is H, these materials are polymers of ethylene oxide, which are also known as polyethylene oxides, polyoxyethylenes, and polyethylene glycols. Other useful polymers include mixed polyethylene-polypropylene glycols, or polyoxyethylene-polyoxypropylene copolymer polymers. Polyethylene glycol polymers useful herein are PEG-2M wherein R⁹⁵ equals H and x3 has an average value of about 2,000 (PEG-2M is also known as Polyox WSR® N-10, which is available from Union Carbide and as PEG-2,000); PEG-5M wherein R⁹⁵ equals H and x3 has an average value of about 5,000 (PEG-5M is also known as Polyox WSR® N-35 and Polyox WSR® N-80, both available from Union Carbide and as PEG-5,000 and Polyethylene Glycol 300,000); PEG-7M wherein R⁹⁵ equals H and x3 has an average value of about 7,000 (PEG-7M is also known as Polyox WSR® N-750 available from Union Carbide); PEG-9M wherein R⁹⁵ equals H and x3 has an average value of about 9,000 (PEG 9-M is also known as Polyox WSR® N-3333 available from Union Carbide); and PEG-14 M wherein R⁹⁵ equals H and x3 has an average value of about 14,000 (PEG-14M is also known as Polyox WSR® N-3000 available from Union Carbide).

Commercially available additional viscosity modifiers highly useful herein include Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxypropyl cellulose with tradename KLUCEL, all supplied by Hercules, hydroxyethyl cellulose with tradename NATROSOL 250HBR and 250MBR available from Aqualon, ethylene oxide and/or propylene oxide based polymers with tradenames CARBOWAX PEGs, POLYOX WASRs, and UCON FLUIDS, all supplied by Amerchol.

### VOLATILE COMPOUND

The compositions of the present invention may contain a volatile compound selected from the group consisting of an isoparaffin hydrocarbon having a boiling point of from about 60 to about 260°C, a volatile silicone compound having from 2 to 7 silicon atoms, and mixtures thereof. The volatile silicone is preferably used in the present compositions of the present invention, and more preferably, a volatile cyclic silicone compound is used in the compositions of the present invention. The volatile compound useful herein is believed to reduce sticky and greasy feeling, and leave the hair and hands with a clean feeling.

The volatile compound can be selected according to the compatibility with other components, and other desired characteristic of the composition of the present invention, for example, can be included in the composition of the present invention at a level by weight of preferably from about 1% to about 70%, more preferably from about 1% to about 60%, still preferably from about 1% to about 50%.

The volatile isoparaffin hydrocarbons useful herein have a boiling point of from about 60 to about 260°C. Commercially available volatile isoparaffin hydrocarbons useful herein include Isopar® series available from Exxon Chemical, Shellsol series available from Shell.

The volatile silicone compounds useful herein include polyalkyl or polyaryl siloxanes with the following structure (I): 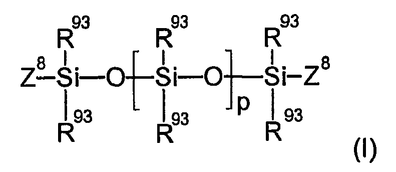 wherein R⁹³ is independently alkyl or aryl, and x is an integer from about 0 to about 5. Z⁸ represents groups which block the ends of the silicone chains. Preferably, R⁹³ groups include methyl, ethyl, propyl, phenyl, methylphenyl and phenylmethyl, Z⁸ groups include hydroxy, methyl, methoxy, ethoxy, propoxy, and aryloxy. More preferably, R⁹³ groups and Z⁸ groups are methyl groups. The preferred volatile silicone compounds are hexamethyldisiloxane, octamethyltrisiloxane, decamethyltetrasiloxane, hexadecamethylheptasiloxane. Commercially available volatile silicone compounds useful herein include octamethyltrisiloxane with tradename SH200C-1cs, decamethyltetrasiloxane with tradename SH200C-1.5cs, hexadecamethylheptasiloxane with tradename SH200C-2cs, all available from Dow Corning.

The volatile silicone compounds useful herein also include a cyclic silicone compound having the formula: 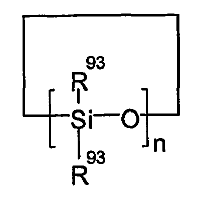 wherein R⁹³ is independently alkyl or aryl, and n is an integer of from 3 to 7. Preferably, R⁹³ groups include methyl, ethyl, propyl, phenyl, methylphenyl and phenylmethyl. More preferably, R⁹³ groups are methyl groups. The preferred volatile silicone compounds are octamethylcyclotetrasiloxane, decamethylcyclopentasiloxane, tetradecamethylcyclohexasiloxane. Commercially available volatile silicone compounds useful herein include octamethylcyclotetrasiloxane with tradename SH244, decamethylcyclopentasiloxane with tradename DC 345 all available from Dow Corning.

### VISIBLE PARTICLE

The compositions of the present invention may further contain a visible particle. By definition, a visible particle is a particle which can be distinctively detected as an individual particle by the naked eye when comprised in the present composition, and which is stable in the present composition. The visible particle can be of any size, shape, or color, according to the desired characteristic of the product, so long as it is distinctively detected as an individual particle by the naked eye. Generally, the visible particle has an average diameter of from about 50µm to about 3000µm, preferably from about 100µm to about 1000µm, more preferably from about 300µm to about 1000µm. By stable, it is meant that the visible particles are not disintegrated, agglomerated, or separated under normal shelf conditions. In one preferred embodiment of the present invention, the composition is substantially transparent. In such an embodiment, the visible particles provide a highly suitable aesthetic benefit. What is generally meant by transparent, is that a black substance having the size of a 1 cm X 1 cm square can be detected by the naked eye through 1 cm thickness of the present composition.

The visible particles herein are used at levels of from about 0.01 % to about 5% by weight of the composition.

The visible particle herein comprises a structural material and preferably an encompassed material.

The structural material provides a certain strength to the visible particle so that they retain their distinctively detectable structure in the present composition under normal shelf conditions. In one preferred embodiment, the structural material further can be broken and disintegrated with very little shear on the hand with the fingers upon use.

Visible particles useful herein include capsules, shelled particles, beads, pellets, droplets, pills, caplets, tablets, grains, flakes, powders and granules. The visible particles can be solid or liquid, filled or un-filled, so long as they are stable in the present composition. The structural material used for making the visible particles varies depending on the compatibility with other components, as well as material, if any, to be encompassed in the visible particles. Exemplary materials for making the visible particles herein include: polysaccharide and saccharide derivatives such as crystalline cellulose, cellulose acetate, cellulose acetate butyrate, cellulose acetate phthalate, cellulose nitrate, ethylcellulose, hydroxypropylcellulose, hydroxypropylmethylcellulose, hydroxypropylmethylcellulose phthalate, methyl cellulose, sodium carboxymethylcellulose, gum acacia (gum arabic), agar, agarose, maltodextrin, sodium alginate, calcium alginate, dextran, starch, galactose, glucosamine, cyclodextrin, chitin, amylose, amylopectin, glycogen, laminaran, lichenan, curdlan, inulin, levan, pectin, mannan, xylan, alginic acid, arabic acid, glucommannan, agarose, agaropectin, prophyran, carrageenen, fucoidan, glycosaminoglycan, hyaluronic acid, chondroitin, peptidoglycan, lipopolysaccharide, guar gum, starch, and starch derivatives; oligosaccharides such as sucrose, lactose, maltose, uronic acid, muramic acid, cellobiose, isomaltose, planteose, melezitose, gentianose, maltotriose, stachyose, glucoside and polyglucoside; monosaccharides such as glucose, fructose, and mannose; synthetic polymers such as acrylic polymers and copolymers including polyacrylamide, poly(alkyl cyanoacrylate), and poly(ethylene-vinyl acetate), and carboxyvinyl polymer, polyamide, poly(methyl vinyl ether-maleic anhydride), poly(adipyl-L-lysine), polycarbonate, polyterephthalamide, polyvinyl acetate phthalate, poly(terephthaloyl-L-lysine), polyarylsulfone, poly(methylmethacrylate), poly(ε-caprolactone), polyvinylpyrrolidone, polydimethylsiloxane, polyoxyethylene, polyester, polyglycolic acid, polylactic acid, polyglutamic acid, polylysine, polystyrene, poly(styrene-acrylonitrile), polyimide, and poly(vinyl alcohol); and other material such as fat, fatty acid, fatty alcohol, milk solids, molasses, gelatin, gluten, albumin, shellac, caseinate, bees wax, carnauba wax, spermaceti wax, hydrogenated tallow, glycerol monopalmitate, glycerol dipalmitate, hydrogenated castor oil, glycerol monostearate, glycerol distearate, glycerol tristearate, 12-hydroxystearyl alcohol, protein, and protein derivatives; and mixtures thereof. Components herein may be described in other sections as useful components for the present composition. The components herein, however, are substantially used to make the structure of the visible particles, and are not dissolved or dispersed in the bulk of the present composition under normal shelf conditions.

Highly preferable structural material herein comprises components selected from the group consisting of polysaccharides and their derivatives, saccharides and their derivatives, oligosaccharides, monosaccharides, and mixtures thereof, still preferably, components from the above mentioned group wherein components having various water solubility are selected. In a particularly preferred embodiment, the structural material is made of components selected from the group consisting of cellulose, cellulose derivatives, saccharides, and mixtures thereof.

The visible particle herein may encompass, contain, or be filled with an encompassed material. Such encompassed material can be water soluble or water insoluble, and comprise components such as: vitamins, amino acids, proteins and protein derivatives, herbal extracts, pigments, dyes, antimicrobial agents, chelating agents, UV absorbers, optical brighteners, silicone compounds, perfumes, humectants which are generally water soluble, additional conditioning agents which are generally water insoluble, and mixtures thereof. In one embodiment, water soluble components are preferred encompassed material. In another embodiment, components selected from the group consisting of vitamins, amino acids, proteins, protein derivatives, herbal extracts, and mixtures thereof are preferred encompassed material. In yet another embodiment, components selected from the group consisting of vitamin E, panthenyl ethyl ether, panthenol, Polygonum multiflori extracts, and mixtures thereof are preferred encompassed material.

Vitamins and amino acids useful as encompassed material herein include: water soluble vitamins such as vitamin B1, B2, B6, B12, C, pantothenic acid, panthenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine , tryptamine, and their salts.

Pigments useful as encompassed material herein include inorganic, nitroso, monoazo, bisazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names: Acid Red 18, 26, 27,33, 51, 52, 87, 88, 92, 94, 95, Acid Yellow 1, 3, 11, 23, 36, 40, 73, Food Yellow 3, Food Green 3, Food blue 2, Food Red 1, 6, Acid Blue 5, 9, 74, Pigment Red 57-1, 53(Na), Basic Violet 10, Solvent Red 49, Acid orange 7, 20, 24, Acid Green 1, 3, 5, 25, Solvent Green 7, Acid Violet 9, 43; water insoluble components such as those having C. I. Names: Pigment Red 53(Ba), 49(Na), 49(Ca), 49(Ba), 49(Sr), 57, Solvent Red 23, 24, 43, 48, 72, 73, Solvent Orange 2, 7, Pigment Red 4, 24, 48, 63(Ca)3, 64, Vat Red 1, Vat blue 1, 6, Pigment Orange 1, 5, 13, Solvent Yellow 5, 6, 33, Pigment Yellow 1, 12, Solvent Green 3, Solvent Violet 13, Solvent Blue 63, Pigment Blue 15, titanium dioxides, chlorophyllin copper complex, ultramarines, aluminum powder, bentonite, calcium carbonate, barium sulfate, bismuthine, calcium sulfate, carbon black, bone black, chromic acid, cobalt blue, gold, ferric oxides, hydrated ferric oxide, ferric ferrocyanide, magnesium carbonate, manganous phosphate, silver, and zinc oxides.

Antimicrobial agents useful as encompassed material include those useful as cosmetic biocides and antidandruff agents including: water soluble components such as piroctone olamine, water insoluble components such as 3,4,4'- trichlorocarbanilide (trichlosan), trichlocarban and zinc pyrithione. Chelating agents useful as encompassed material include: 2,2'-dipyridylamine; 1,10-phenanthroline {o-phenanthroline}; di-2-pyridyl ketone; 2,3-bis(2-pyridyl) pyrazine; 2,3-bis(2-pyridyl)-5,6-dihydropyrazine; 1,1'-carbonyldiimidazole; 2,4-bis(5,6-diphenyl-1,2,4-triazine-3-yl)pyridine; 2,4,6-tri(2-pyridyl)-1,3,5-triazine; 4,4'-dimethyl-2,2'dipyridyl; 2,2'-biquinoline; di-2-pyridyl glyoxal {2,2'-pyridil}; 2-(2-pyridyl)benzimidazole; 2,2'-bipyrazine; 3-(2-pyridyl)5,6-diphenyl-1,2,4-trazine; 3-(4-phenyl-2-pyridyl)-5-phenyl-1,2,4-triazine; 3-(4-phenyl-2-pyridyl)-5,6-diphenyl-1,2,4-triazine; 2,3,5,6-tetrakis-(2'-pyridyl)-pyrazine; 2,6-pyridinedicarboxylic acid; 2,4,5-trihydroxypyrimidine; phenyl 2-pyridyl ketoxime; 3-amino-5,6-dimethyl-1,2,4-triazine; 6-hydroxy-2-phenyl-3(2H)-pyridazinone; 2,4-pteridinediol {lumazine}; 2,2-'dipyridyl; and 2,3-dihydroxypyridine.

Useful silicone compounds, humectants, additional conditioning agents, UV absorbers, optical brighteners, and herbal extracts for encompassed material are the same as those exemplified in other portions of the specification. The components herein, however, are substantially retained within the breakable visible particles, and are substantially not dissolved in the bulk of the present composition under normal shelf conditions.

Particularly useful commercially available visible particles herein are those with tradenames Unisphere and Unicerin available from Induchem AG (Switzerland), and Confetti Dermal Essentials available from United-Guardian Inc. (NY, USA). Unisphere and Unicerin particles are made of microcrystalline cellulose, hydroxypropyl cellulose, lactose, vitamins, pigments, and proteins. Upon use, the Unisphere and Unicerin particles can be disintegrated with very little shear on the hand with the fingers with practically no resistance, and readily dissolve in the composition.

### UV ABSORBER

The compositions of the present invention may further contain a UV (ultraviolet) absorber. UV absorbers are particularly useful for compositions of the present invention which are substantially transparent. The UV absorbers herein are preferably used at levels by weight of the composition of from about 0.01 % to about 10%.

UV absorbers useful herein can be water soluble or water insoluble, including: p-aminobenzoic acid, its salts and its derivatives (ethyl, isobutyl, glyceryl esters; p-dimethylaminobenzoic acid); anthranilates (i.e., o-aminobenzoates; methyl, menthyl, phenyl, benzyl, phenylethyl, linalyl, terpinyl, and cyclohexenyl esters); salicylates (amyl, phenyl, benzyl, menthyl, glyceryl, and dipropyleneglycol esters); cinnamic acid derivatives (menthyl and benzyl esters, -phenyl cinnamonitrile; butyl cinnamoyl pyruvate; trihydroxycinnamic acid derivatives (esculetin, methylesculetin, daphnetin, and the glucosides, esculin and daphnin); dibenzalacetone and benzalacetophenone; naphtholsulfonates (sodium salts of 2-naphthol-3,6-disulfonic and of 2-naphthol-6,8-disulfonic acids); dihydroxy-naphthoic acid and its sals; o-and p-Hydroxybiphenyldisulfonates; quinine salts (bisulfate, sulfate, chloride, oleate, and tannate); quinoline derivatives (8-hydroxyquinoline salts, 2-phenylquinoline); hydroxy- or methoxy-substituted benzophenones; uric and vilouric acids; tannic acid and its derivatives (e.g., hexaethylether); (butyl carbityl) (6-propyl piperonyl) ether; hydroquinone; benzophenones (oxybenzene, sulisobenzone, dioxybenzone, benzoresorcinol, 2,2',4,4'-Tetrahydroxybenzophenone, 2,2'-Dihydroxy-4,4'-dimethoxybenzophenone, octabenzone); 4-lsopropyldibenzoylmethane; butylmethoxydibenzoylmethane; etocrylene; and 4-isopropyl-di-benzoylmethane. Of these, 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone ethyl 4-[bis(hydroxypropyl)]aminobenzoate, 2-ethylhexyl2-cyano-3,3-diphenylacrylate, 2-ethylhexylsalicylate, glyceryl p-aminobenzoate, 3,3,5-trimethylcyclohexylsalicylate, methylanthranilate, p-dimethyl-aminobenzoic acid or aminobenzoate, 2-ethylhexyl p-dimethylamino-benzoate, 2-phenylbenzimidazole-5-sulfonic acid, 2-(p-dimethylaminophenyl)-5-sulfonicbenzoxazoic acid and mixtures thereof. Preferred sunscreens useful in the compositions of the present invention are 2-ethylhexyl p-methoxycinnamate, butylmethoxydibenzoylmethane, 2-hydroxy-4-methoxybenzophenone, octyldimethyl p-aminobenzoic acid and mixtures thereof.

### HERBAL EXTRACT

The compositions of the present invention may further contain herbal extracts. Herbal extracts useful herein include those which are water soluble and those which are water insoluble. Useful herbal extracts herein include: Polygonum multiflori Extract, Houttuynia cordate extract, Phellodendron Bark extract, melilot extract, white dead nettle extract, licorice root extract, herbaceous peony extract, soapwort extract, dishcloth gourd extract, cinchona extract, creeping saxifrage extract, Sophora angustifolia extract, candock extract, common fennel extract, primrose extract, rose extract, Rehmannia glutinosa extract, lemon extract, shikon extract, alloe extract, iris bulb extract, eucalyptus extract, field horsetail extract, sage extract, thyme extract, tea extract, laver extract, cucumber extract, clove extract, raspberry extract, melissa extract, ginseng extract, carrot extract, horse chestnut extract, peach extract, peach leaf extract, mulberry extract, cornflower extract, hamamelis extract, placenta extract, thymus extract, silk extract, algae extract, althea extract, angelica dahurica extract, apple extract, apricot kernel extract, arnica extract, Artemisia capillaris extract, astragal extract, balm mint extract, perilla extract, birch bark extract, bitter orange peel extract, Thea sinensis extract, burdock root extract, burnet extract, butcherbroom extract, Stephania cepharantha extract, matricaria extract, chrysanthemum flower extract, citrus unshiu peel extract, cnidium extract, coix seed extract, coltsfoot extract, comfrey leaf extract, crataegus extract, evening primrose oil, gambir extract, ganoderma extract, gardenia extract, gentian extract, geranium extract, ginkgo extract, grape leaf extract, crataegus extract, henna extract, honeysuckle extract, honeysuckle flower extract, hoelen extract, hops extract, horsetail extract, hydrangea extract, hypericum extract, isodonis extract, ivy extract, Japanese angelica extract, Japanese coptis extract, juniper extract, jujube extract, lady's mantle extract, lavender extract, lettuce extract, licorice extract, linden extract, lithospermum extract, loquat extract, luffa extract, malloti extract, mallow extract, calendula extract, moutan bark extract, mistletoe extract, mukurossi extract, mugwort extract, mulberry root extract, nettle extract, nutmeg extract, orange extract, parsely extract, hydrolyzed conchiorin protein, peony root extract, peppermint extract, philodendron extract, pine cone extract, platycodon extract, polygonatum extract, rehmannia extract, rice bran extract, rhubarb extract, rose fruit extract, rosemary extract, royal jelly extract, safflower extract, saffron crocus extract, sambucus extract, saponaria extract, Sasa albo marginata extract, Saxifraga stolonifera extract, scutellaria root extract, Cortinellus shiitake extract, lithospermum extract, sophora extract, laurel extract, calamus root extract, swertia extract, thyme extract, linden extract, tomato extract, turmeric extract, uncaria extract, watercress extract, logwood extract, grape extract, white lily extract, rose hips extract, wild thyme extract, witch hazel extract, yarrow extract, yeast extract, yucca extract, zanthoxylum extract, and mixtures thereof.

Commercially available herbal extracts useful herein include Polygonum multiflori extracts which are water soluble, and available from Institute of Occupational Medicine, CAPM, China National Light Industry, and Maruzen, and other herbal extracts listed above available from Maruzen.

### ADDITIONAL CONDITIONING AGENT

The compositions of the present invention may further contain an additional conditioning agent selected from the group consisting of high melting point compounds, high molecular weight ester oils, additional oily compounds, and mixtures thereof. Additional conditioning agents are selected according to the compatibility with other components, and the desired characteristic of the product. The additional conditioning agents herein are preferably used at levels by weight of the composition of from about 0.01 % to about 10%.

### High melting point compound

The high melting point compound useful herein have a melting point of at least about 25°C selected from the group consisting of fatty alcohols, fatty acids, fatty alcohol derivatives, fatty acid derivatives, hydrocarbons, steroids, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than about 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

It is believed that these high melting point compounds cover the hair surface and reduce friction, thereby resulting in providing smooth feel on the hair and ease of combing.

The high melting point compound is preferably included in the composition at a level by weight of from about 0.01 % to about 5%, more preferably from about 0.1 % to about 1 %.

The fatty alcohols useful herein are those having from about 14 to about 30 carbon atoms, preferably from about 16 to about 22 carbon atoms. These fatty alcohols can be straight or branched chain alcohols and can be saturated or unsaturated. Nonlimiting examples of fatty alcohols include, cetyl alcohol, stearyl alcohol, behenyl alcohol, and mixtures thereof.

The fatty acids useful herein are those having from about 10 to about 30 carbon atoms, preferably from about 12 to about 22 carbon atoms, and more preferably from about 16 to about 22 carbon atoms. These fatty acids can be straight or branched chain acids and can be saturated or unsaturated. Also included are diacids, triacids, and other multiple acids which meet the requirements herein. Also included herein are salts of these fatty acids. Nonlimiting examples of fatty acids include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, *i.e.* a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

Hydrocarbons useful herein include compounds having at least about 20 carbons.

Steroids useful herein include compounds such as cholesterol.

High melting point compounds of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least about 90%, preferably at least about 95%. These single compounds of high purity provide good rinsability from the hair when the consumer rinses off the composition.

Commercially available high melting point compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago Illinois, USA), HYSTRENE available from Witco Corp. (Dublin Ohio, USA), and DERMA available from Vevy (Genova, Italy); and cholesterol having tradename NIKKOL AGUASOME LA available from Nikko.

### High Molecular Weight Ester Oils

High molecular weight ester oils are useful herein. The high molecular weight ester oils useful herein are those which are water insoluble, have a molecular weight of at least about 500, preferably at least about 800, and are in liquid form at 25°C. Useful high molecular weight ester oils herein include trimethylol ester oils, poly α-olefin oils, citrate ester oils, glyceryl ester oils, and mixtures thereof. As used herein, the term "water insoluble" means the compound is substantially not soluble in water at 25°C; when the compound is mixed with water at a concentration by weight of above 1.0%, preferably at above 0.5%, the compound is temporarily dispersed to form an unstable colloid in water, then is quickly separated from water into two phases.

The high molecular weight ester oil herein provides conditioning benefits such as moisturized feel, smooth feel, and manageability control to the hair when the hair is dried, yet does not leave the hair feeling greasy. It is believed that water insoluble oily material in general are capable of being deposited on the hair. Without being bound by theory, it is believed that, because of its bulkiness, the high molecular weight ester oil covers the surface of the hair and, as a result, the high molecular weight ester oil reduces hair friction to deliver smoothness and manageability control to the hair. It is also believed that, because it has some hydrophilic groups, the high molecular weight ester oil provides moisturized feel, yet, because it is liquid, does not leave the hair feeling greasy. The high molecular weight ester oil is chemically stable under normal use and storage conditions.

Trimethylol ester oils useful herein are those having the following formula: 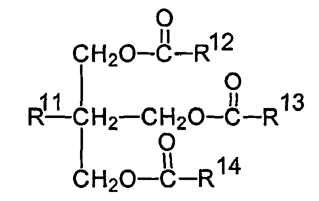 wherein R¹¹ is an alkyl group having from 1 to about 30 carbons, and R¹², R¹³, and R¹⁴, independently, are branched, straight, saturated, or unsaturated alkyl, aryl, and alkylaryl groups having from 1 to about 30 carbons. Preferably, R¹¹ is ethyl and R¹², R¹³, and R¹⁴, independently, are branched, straight, saturated, or unsaturated alkyl groups having from 8 to about 22 carbons. More preferably, R¹¹, R¹², R¹³ and R¹⁴ are defined so that the molecular weight of the compound is from about 800 to about 1200.

Poly α-olefin oils useful herein are those having the following formula and having a viscosity of from about 1 to about 35,000 cst, a molecular weight of from about 200 to about 60,000, and a polydispersity of no more than about 3; 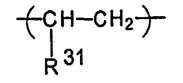 wherein R³¹ is an alkyl having from about 4 to 14 carbons, preferably 4 to 10 carbons. Poly α-olefin oils having a molecular weight of at least about 800 are useful herein. Such high molecular weight poly α-olefin oils are believed to provide long lasting moisturized feel to the hair. Poly α-olefin oils having a molecular weight of less than about 800 are useful herein. Such low molecular weight poly α-olefin oils are believed to provide a smooth, light, clean feel to the hair.

Citrate ester oils useful herein are those having a molecular weight of at least about 500 having the following formula: 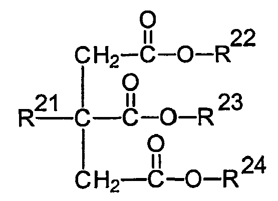 wherein R²¹ is OH or CH₃COO, and R²², R²³, and R²⁴, independently, are branched, straight, saturated, or unsaturated alkyl, aryl, and alkylaryl groups having from 1 to about 30 carbons. Preferably, R²¹ is OH, and R²², R²³, and R²⁴, independently, are branched, straight, saturated, or unsaturated alkyl, aryl, and alkylaryl groups having from 8 to about 22 carbons. More preferably, R²¹, R²², R²³ and R²⁴ are defined so that the molecular weight of the compound is at least about 800.

Glyceryl ester oils useful herein are those having a molecular weight of at least about 500 and having the following formula: 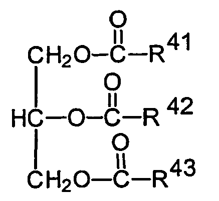 wherein R⁴¹, R⁴², and R⁴³, independently, are branched, straight, saturated, or unsaturated alkyl, aryl, and alkylaryl groups having from 1 to about 30 carbons. Preferably, R⁴¹, R⁴², and R⁴³, independently, are branched, straight, saturated, or unsaturated alkyl, aryl, and alkylaryl groups having from 8 to about 22 carbons. More preferably, R⁴¹, R⁴², and R⁴³ are defined so that the molecular weight of the compound is at least about 800.

Particularly useful trimethylol ester oils herein include trimethylolpropane triisostearate, trimethylolpropane trioleate, and mixtures thereof. Such compounds are available from Shin-nihon Rika with tradenames PTO, ENUJERUBU TP3SO.

Particularly useful poly α-olefin oils herein include polydecenes with tradenames PURESYN 6 having a number average molecular weight of about 500 and PURESYN 100 having a number average molecular weight of about 3000 and PURESYN 300 having a number average molecular weight of about 6000 available from Mobil Chemical Co.

Particularly useful citrate ester oils herein include triisocetyl citrate with tradename CITMOL 316 available from Bernel, triisostearyl citrate with tradename PELEMOL TISC available from Phoenix, and trioctyldodecyl citrate with tradename CITMOL 320 available from Bernel.

Particularly useful glyceryl ester oils herein include triisostearin with tradename SUN ESPOL G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks.

### Additional Oily Compounds

Additional oily compounds useful herein include fatty alcohols and their derivatives, fatty acids and their derivatives, and hydrocarbons. The additional oily compounds useful herein may be volatile or nonvolatile, and have a melting point of not more than about 25°C. Without being bound by theory, it is believed that, the additional oily compounds may penetrate into the hair to modify the hydroxy bonds of the hair, thereby resulting in providing softness and flexibility to the hair. The additional oily compounds of this section are to be distinguished from the high melting point compounds described above. Nonlimiting examples of the additional oily compounds are found in International Cosmetic Ingredient Dictionary, Fifth Edition, 1993, and CTFA Cosmetic Ingredient Handbook, Second Edition, 1992.

The fatty alcohols useful herein include those having from about 10 to about 30 carbon atoms, preferably from about 12 to about 22 carbon atoms, and more preferably from about 16 to about 22 carbon atoms. These fatty alcohols can be straight or branched chain alcohols and can be saturated or unsaturated alcohols, preferably unsaturated alcohols. Nonlimiting examples of these compounds include oleyl alcohol, palmitoleic alcohol, isostearyl alcohol, isocetyl alchol, undecanol, octyl dodecanol, octyl decanol, octyl alcohol, caprylic alcohol, decyl alcohol and lauryl alcohol.

The fatty acids useful herein include those having from about 10 to about 30 carbon atoms, preferably from about 12 to about 22 carbon atoms, and more preferably from about 16 to about 22 carbon atoms. These fatty acids can be straight or branched chain acids and can be saturated or unsaturated. Suitable fatty acids include, for example, oleic acid, linoleic acid, isostearic acid, linolenic acid, ethyl linolenic acid, ethyl linolenic acid, arachidonic acid, and ricinolic acid.

The fatty acid derivatives and fatty alcohol derivatives are defined herein to include, for example, esters of fatty alcohols, alkoxylated fatty alcohols, alkyl ethers of fatty alcohols, alkyl ethers of alkoxylated fatty alcohols, and bulky ester oils such as trimethylol ester oils, citrate ester oils, glyceryl ester oils, and mixtures thereof. Nonlimiting examples of fatty acid derivatives and fatty alcohol derivatives, include, for example, methyl linoleate, ethyl linoleate, isopropyl linoleate, isodecyl oleate, isopropyl oleate, ethyl oleate, octyldodecyl oleate, oleyl oleate, decyl oleate, butyl oleate, methyl oleate, octyldodecyl stearate, octyldodecyl isostearate, octyldodecyl isopalmitate, octyl isopelargonate, octyl pelargonate, hexyl isostearate, isopropyl isostearate, isodecyl isononanoate, isopropyl stearate, ethyl stearate, methyl stearate and Oleth-2. Bulky ester oils such as pentaerythritol ester oils, trimethylol ester oils, citrate ester oils and glyceryl ester oils useful herein are those which have a molecular weight of less than about 800, preferably less than about 500.

The hydrocarbons useful herein include straight chain, cyclic, and branched chain hydrocarbons which can be either saturated or unsaturated, so long as they have a melting point of not more than about 25°C. These hydrocarbons have from about 12 to about 40 carbon atoms, preferably from about 12 to about 30 carbon atoms, and preferably from about 12 to about 22 carbon atoms. Also encompassed herein are polymeric hydrocarbons of alkenyl monomers, such as polymers of C₂₋₆ alkenyl monomers. These polymers can be straight or branched chain polymers. The straight chain polymers will typically be relatively short in length, having a total number of carbon atoms as described above. The branched chain polymers can have substantially higher chain lengths. The number average molecular weight of such materials can vary widely, but will typically be up to about 500, preferably from about 200 to about 400, and more preferably from about 300 to about 350. Also useful herein are the various grades of mineral oils. Mineral oils are liquid mixtures of hydrocarbons that are obtained from petroleum. Specific examples of suitable hydrocarbon materials include paraffin oil, mineral oil, dodecane, isododecane, hexadecane, isohexadecane, eicosene, isoeicosene, tridecane, tetradecane, polybutene, polyisobutene, and mixtures thereof. Preferred for use herein are hydrocarbons selected from the group consisting of mineral oil, poly α-olefin oils such as isododecane, isohexadecane, polybutene, polyisobutene, and mixtures thereof.

Commercially available fatty alcohols and their derivatives useful herein include: oleyl alcohol with tradename UNJECOL 90BHR available from Shin Nihon Rika, various liquid esters with tradenames SCHERCEMOL series available from Scher, and hexyl isostearate with a tradename HIS and isopropryl isostearate having a tradename ZPIS available from Kokyu Alcohol. Commercially available bulky ester oils useful herein include: trimethylolpropane tricaprylate/tricaprate with tradename MOBIL ESTER P43 from Mobil Chemical Co. Commercially available hydrocarbons useful herein include isododecane, isohexadecane, and isoeicosene with tradenames PERMETHYL 99A, PERMETHYL 101A, and PERMETHYL 1082, available from Presperse (South Plainfield New Jersey, USA), a copolymer of isobutene and normal butene with tradenames INDOPOL H-100 available from Amoco Chemicals (Chicago Illinois, and USA), mineral oil with tradename BENOL available from Witco, isoparaffin with tradename ISOPAR from Exxon Chemical Co. (Houston Texas, USA.)

### Other Additional Components

The compositions of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as glutamic acid, citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate, antidandruff agents such as zinc pyrithione; and mixtures thereof.

### PRODUCT FORMS

The hair conditioning compositions of the present invention can be in the form of rinse-off products or leave-on products, can be transparent or opaque, and can be formulated in a wide variety of product forms, including but not limited to creams, gels, emulsions, mousses and sprays. Preferably, the compositions of the present invention are in the form of leave-on products.

### EXAMPLES

The following examples further describe and demonstrate embodiments within the scope of the present invention. Ingredients are identified by chemical or CTFA name, or otherwise defined below.

All percentages herein are based upon the total weight of the compositions of the present invention unless otherwise specified. All such weight percentages as they pertain to listed ingredients are based on the active level and, therefore, do not include carriers or by-products that may be included in commercially available materials.

### Compositions (wt%)

| | Ex.1 | Ex.2 | Ex. 3 | Ex.4 |
|---|---|---|---|---|
| Acrylic acid/Alkyl acrylate copolymer *1 | 0.35 | 0.5 | 0.5 | 0.1 |
| PEG-modified glyceride *2 | 4.0 | - | 8.0 | 2.0 |
| PEG-modified glyceryl fatty acid ester *3 | - | 4.0 | - | - |
| Dimethicone copolyol-1 *4 | - | - | - | - |
| Dimethicone copolyol-2 *5 | - | 4.0 | - | - |
| Polypropylene glycol *6 | - | - | - | 2.0 |
| Pentaerythritol ester oil *7 | - | - | - | 2.0 |
| Dimethicone/Dimethiconol *8 | 1.0 | - | 1.0 | 0.5 |
| Cyclomethicone/Dimethiconol *9 | - | - | - | - |
| Cyclomethicone/Dimethicone *10 | - | 3.0 | - | - |
| Propylene Glycol *11 | - | - | - | 2.0 |
| Polyethylene Glycol 200 *12 | 2.0 | 2.0 | 4.0 | 1.0 |
| Polyquaternium-39 *13 | 0.2 | 0.1 | - | 1.0 |
| Polyquaternium-47 *14 | - | - | 0.1 | - |
| Carbomer *15 | 0.1 | 0.1 | 0.1 | 0.5 |
| Polygonum multiflori extract *16 | - - | - | - | 0.1 |
| Visible particles *17 | 0.1 | 0.1 | 0.1 | 0.1 |
| Vitamin E *18 | - | - | 0.05 | 0.05 |
| Panthenol *19 | - | - | 0.1 | 0.1 |
| Benzophenone-4 *20 | 0.05 | 0.1 | 0.1 | - |
| Octyl Methoxycinnamate *21 | - | - | - | 0.2 |
| Methyl Paraben | 0.2 | 0.2 | 0.2 | 0.2 |
| Phenoxyethanol | 0.3 | 0.3 | 0.3 | 0.3 |
| Disodium EDTA | 0.13 | 0.1 | 0.1 | 0.1 |
| Perfume solution | 0.05 | 0.1 | 0.1 | 0.1 |
| Deionized Water | -------------------- q.s. to 100% -------------------- | | | |

| | Ex.5 | Ex.6 | Ex.7 | Ex.8 | Ex.9 | Ex.10 |
|---|---|---|---|---|---|---|
| Acrylates copolymer *22 | 0.3 | 0.4 | 0.8 | - | - | - |
| Crosslinked polymer *23 | - | - | - | 0.3 | 0.1 | 0.5 |
| Propylene Glycol Dicaprylate/Dicaprate *23 | - | - | - | 0.3 | 0.1 | 0.5 |
| PEG-modified glyceride *2 | 8.0 | 4.0 | - | 4.0 | - | 2.0 |
| PEG-modified glyceryl fatty acid ester *3 | - | - | 4.0 | - | 4.0 | - |
| Dimethicone copolyol-1 *4 | - | 4.0 | - | - | - | - |
| Dimethicone copolyol-2 *5 | - | - | - | - | 4.0 | - |
| Polypropylene glycol *6 | - | - | 2.0 | - | - | 2.0 |
| Pentaerythritol ester oil *7 | - | - | 2.0 | - | - | 2.0 |
| Distearyldimonium chloride *24 | 0.8 | 1.0 | - | 0.8 | - | 0.8 |
| Stearamidopropyl Dimethylamine *25 | - | - | 0.8 | - | 1.2 | - |
| Dimethicone/Dimethiconol *8 | 1.0 | - | 0.5 | 1.0 | - | 0.5 |
| Cyclomethicone/Dimethiconol *9 | - | 3.0 | - | - | 3.0 | - |
| Cyclomethicone/Dimethicone *10 | - | - | 3.0 | - | - | 3.0 |
| Propylene Glycol *11 | - | - | 2.0 | - | 2.0 | 2.0 |
| Polyethylene Glycol 200 *12 | 2.0 | 2.0 | 1.0 | 4.0 | - | 1.0 |
| Polyquaternium-39 *13 | - | 0.2 | 0.1 | - | 0.5 | 1.0 |
| Polyquaternium-47 *14 | - | - | - | 0.1 | - | - |
| Glutamic acid | 0.12 | 0.12 | 0.1 | 0.1 | 0.1 | 0.1 |
| Cetyl Alcohol *26 | - | - | - | - | 0.1 | - |
| Stearyl Alcohol *27 | - | - | 0.5 | - | - | 0.5 |
| Hydroxyethyl cellulose *28 | - | - | 0.1 | - | - | 0.1 |
| Polygonum multiflori extract *16 | - | - | - | - | 0.1 | 0.1 |
| Visible particles *17 | 0.1 | 0.1 | 0.1 | 0.1 | 0.1 | 0.1 |
| Vitamin E *18 | - | 0.05 | - | 0.05 | 0.05 | 0.05 |
| Panthenol *19 | - | 0.1 | - | 0.1 | 0.1 | 0.1 |
| Benzophenone-4 *20 | 0.05 | 0.1 | 0.1 | 0.1 | 0.1 | - |
| Octyl Methoxycinnamate *21 | - | - | - | - | - | 0.2 |
| Methyl Paraben | 0.2 | 0.2 | 0.2 | 0.2 | 0.2 | 0.2 |
| Phenoxyethanol | 0.3 | 0.3 | 0.3 | 0.3 | 0.3 | 0.3 |
| Disodium EDTA | 0.13 | 0.13 | 0.1 | 0.1 | 0.1 | 0.1 |
| Perfume solution | 0.05 | 0.05 | 0.1 | 0.1 | 0.1 | 0.1 |
| Deionized Water | -------------------- q.s. to 100% -------------------- | | | | | |

| | Ex.11 | Ex.12 | Ex.13 | Ex.14 | Ex.15 | Ex.16 |
|---|---|---|---|---|---|---|
| Hydrophobically modified cellulose ether *29 | 0.45 | 0.5 | - | 0.5 | 1.0 | 0.1 |
| Acrylates copolymer *22 | 0.3 | 0.3 | 0.3 | 0.1 | 0.1 | - |
| Crosslinked polymer *23 | 0.2 | 0.3 | 0.2 | - | 0.1 | 0.5 |
| Propylene Glycol Dicaprylate/Dicaprate *23 | 0.2 | 0.3 | 0.2 | 0.5 | 0.1 | 0.5 |
| PEG-modified glyceride *2 | 4.0 | 8.0 | - | 8.0 | - | - |
| PEG-modified glyceryl fatty acid ester *3 | - | - | 4.0 | - | 2.0 | - |
| Dimethicone copolyol-1 *4 | 2.0 | - | - | - | - | 4.0 |
| Dimethicone copolyol-2 *5 | - | - | 4.0 | - | - | - |
| Polypropylene glycol *6 | - | - | - | - | 2.0 | - |
| Pentaerythritol ester oil *7 | - | - | - | - | 2.0 | - |
| Distearyldimonium chloride *24 | 0.8 | 1.0 | - | 0.8 | - | 0.8 |
| Stearamidopropyl Dimethylamine *25 | - | - | 0.8 | - | 1.2 | - |
| Dimethicone/Dimethiconol *8 | 1.0 | 1.0 | 1.0 | - | - | 0.5 |
| Cyclomethicone/Dimethiconol *9 | - | - | - | 1.5 | 3.0 | - |
| Cyclomethicone/Dimethicone *10 | - | - | - | 1.5 | - | - |
| Propylene Glycol *11 | - | - | - | - | 2.0 | 2.0 |
| Polyethylene Glycol 200 *12 | 2.0 | 2.0 | 2.0 | 4.0 | - | 1.0 |
| Polyquatemium-39 *13 | 0.2 | 0.2 | 0.1 | - | 0.5 | 1.0 |
| Polyquaternium-47 *14 | - | - | - | 0.1 | - | - |
| Glutamic acid | 0.12 | 0.12 | 0.1 | 0.1 | 0.1 | 0.1 |
| Cetyl Alcohol *26 | - | - | - | - | 0.1 | - |
| Stearyl Alcohol *27 | - | - | - | - | - | 0.5 |
| Polygonum multiflori extract *16 | - | - | - | - | 0.1 | 0.1 |
| Visible particles *17 | 0.1 | 0.1 | 0.1 | 0.1 | 0.1 | 0.1 |
| Vitamin E *18 | - | 0.05 | - | 0.05 | 0.05 | 0.05 |
| Panthenol *19 | - | 0.1 | - | 0.1 | 0.1 | 0.1 |
| Benzophenone-4 *20 | 0.05 | 0.1 | 0.1 | 0.1 | 0.1 | - |
| Octyl Methoxycinnamate *21 | - | - | - | - | - | 0.2 |
| Methyl Paraben | 0.2 | 0.2 | 0.2 | 0.2 | 0.2 | 0.2 |
| Phenoxyethanol | 0.3 | 0.3 | 0.3 | 0.3 | 0.3 | 0.3 |
| Disodium EDTA | 0.13 | 0.13 | 0.1 | 0.1 | 0.1 | 0.1 |
| Perfume solution | 0.05 | 0.05 | 0.1 | 0.1 | 0.1 | 0.1 |
| Deionized Water | -------------------- q.s. to 100% -------------------- | | | | | |

| | | | | | | |
|---|---|---|---|---|---|---|
| Definitions of Components *1 Acrylic acid/Alkyl acrylate copolymer: PEMULEN TR-1 available from G.F. Goodrich *2 PEG modified glyceride: Tagat TO available from Goldschmidt Chemical *3 PEG modified glyceryl fatty acid ester: Tagat S available from Goldschmidt Chemical *4 Dimethicone copolyol-1: Abil B 8830 available from Goldschmidt Chemical *5 Dimethicone copolyol-2: Abil B 8852 available from Goldschmidt Chemical *6 Polypropylene glycol: New Pol PP-2000 available from Sanyo Kasei *7 Pentaerythritol Tetraisostearate: KAKPTI available from Koukyu Alcohol Kogyo *8 Dimethicone/Dimethiconol: DC-1403 available from Dow Corning *9 Cyclomethicone/Dimethiconol: DCQ2-1401 available from Dow Corning *10 Cyclomethicone/Dimethicone: Gum/Cyclomethicone blend (15:85) available from Shin-Etsu *11 Propylene Glycol: Available from BASF *12 Polyethylene Glycol 200: Carbowax PEG200 available from Union Carbide *13 Polyquaternium-39: Merquat Plus 3330 available from Calgon *14 Polyquatemium-47: Merquat 2001 available from Calgon *15 Carbomer: Carbopol 981 available from G.F. Goodrich *16 Polygonum multiflori extract: Polygonum multiflori extract available from Occupational Medecine, CAPM. *17 Visible particles: Unispheres AGE-527 available from Induchem *18 Vitamin E: Emix-d Available from Eisai *19 Panthenol: Panthenol Available from Roche. *20 Benzophenone-4: Uvnul MS-40 available from BASF *21 Octyl Methoxycinnamate: Parasol MCX available from Roche *22 Acrylates copolymer: Structure plus available from National starch *23 Crosslinked polymer: Polyquaternium 37 sold as Salcare 96 available from Allied Colloids *23 Propylene Glycol Dicaprylate/Dicaprate: sold as Salcare 96 available from Allied Colloids *24 Distearyldimonium chloride, Varisoft TA100 available from Witco *25 Stearamidopropyl Dimethylamine: available from Inolex *26 Cetyl Alcohol: Konol series available from Shinnihon Rika *27 Stearyl Alcohol: Konol series available from Shinnihon Rika *28 Hydroxyethylcellulose: NATROSOL 250HBR available from Aqualon *29 Hydrophobically modified cellulose ether : Polysurf 67 available from Aqualon | | | | | | |

### Method of Preparation

The polymeric materials such as the acrylic acid/alkyl acrylate copolymer, hydrophobically modified cellulose ether, acrylates copolymer, crosslinked polymer, amphoteric conditioning polymer, and additional viscosity modifier, if present, are dispersed in water at room temperature, mixed by vigorous agitation, and heated to 50°C. The high melting point compounds, if included, are added to the mixture with agitation at above 70°C by either melting such components or by dissolving such components. Then the mixture is cooled to below 40°C, and then the remaining components such as frizz control agents, additional frizz control agents, and cationic surfactants, if included, are added to the mixture with agitation.

Examples 1 through 16 are hair conditioning compositions of the present invention which are for leave-on use. These examples have many advantages. For example, they can provide improved frizz control benefit and hair volume-down benefit while retaining good conditioning benefits and good hair feel and appearance, i.e., they can provide improved frizz control benefit and hair volume-down benefit in addition to other conditioning benefits such as smoothness, softness, and reduction of friction, and leave the hair and hands with a clean feeling.

## Claims

1. A hair conditioning composition for leave-on use on the hair comprising:
(1) a thickening agent selected from the group consisting of (i), (ii), (iii), and (iv);
(i) an acrylic acid/alkyl acrylate copolymer having the following formula: 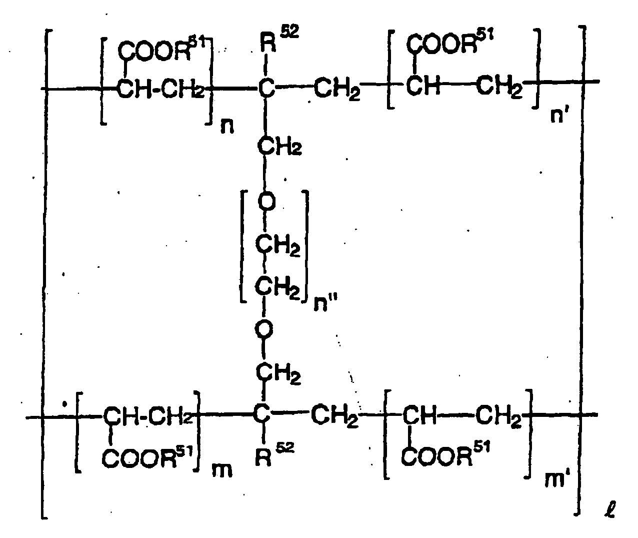 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000;
(ii) an acrylates copolymer comprising by weight:
(a) from about 5% to about 80% of an acrylate monomer selected from the group consisting of a C₁-C₆ alkyl ester of acrylic acid, a C₁-C₆ alkyl ester of methacrylic acid, and mixtures thereof;
(b) from about 5% to about 80% of a monomer selected from the group consisting of a vinyl-substituted heterocyclic compound containing at least one of a nitrogen or sulfur atom, a (meth)acrylamide, a mono- or di-(C₁₋C₄)alkylamino(C₁-C₄)alkyl(meth)acrylate, a mono- or di-(C₁₋C₄)alkylamino(C₁-C₄)alkyl(meth)acrylamide, and mixtures thereof; and
(c) from 0% to about 30% of an associative monomer,
(iii) a crosslinked polymer having the formula (A)ₘ(B)ₙ(C)ₚ, wherein:
(A) is selected from the group consisting of a dialkylaminoalkyl acrylate, a quaternized dialkylaminoalkyl acrylate, an acid addition salt of a quaternized dialkylaminoalkyl acrylate, and mixtures thereof;
(B) is selected from the group consisting of a dialkylaminoalkyl methacrylate, a quaternized dialkylaminoalkyl methacrylate, an acid Addition salt of a quaternized dialkylaminoalkyl methacrylate, and mixtures thereof;
(C) is a nonionic monomer polymerizable with (A) or (B); and
m, n, and p are independently zero or greater, but at least one of m or n is one or greater; and
(iv) mixtures thereof;
(2) a frizz control agent selected from the group consisting of (i), (II), and (III),
(i) PEG-modified glycerides having the structure: 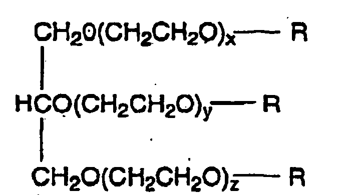 wherein one or more of the R groups is selected from saturated or unsaturated fatty acid moieties derived from animal or vegetable oils wherein the fatty acid moieties have a carbon length chain of from 12 and 22, any other R groups are hydrogen, x; y, z are independently zero or more, the average sum of x+y+z is equal to from about 10 to about 45;
(ii) PEG-modified glyceryl fatty acid esters having the structure: 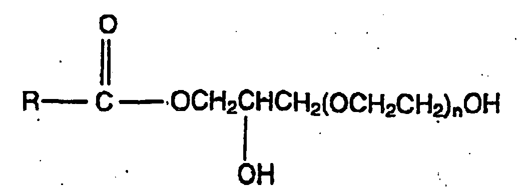 wherein R is an aliphatic group having from 12 to 22 carbon chain length, and n has an average value of from 15 to 30; and
(iii) mixtures thereof; and
(3) an aqueous carrier.

2. The hair conditioning composition according to Claim 1 wherein the sum of x+y+z in the PEG-modified glycerides is equal to from about 20 to 30,

3. The hair conditioning composition according to Claim 1 further comprising dimethicone copolyols selected from the group consisting of (i), (ii), and (iii):
(i) dimethicone copolyols having the structure: 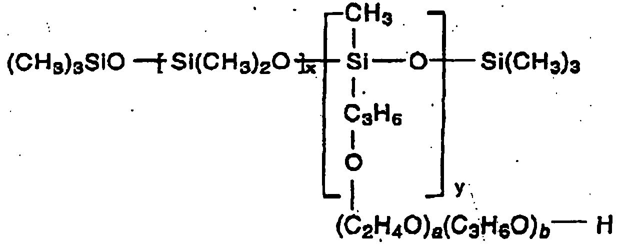 wherein x is an integer from 1 to 2000, y is an integer from 1 to 1000, a is zero or greater, b is zero or greater, the sum of a+b is at least 1, and having an HLB value of about 20 or less;
(ii) dimethicone copolyols having the structure: 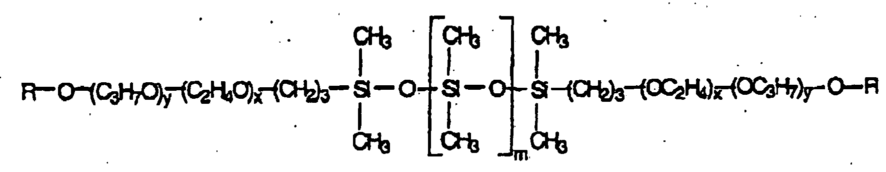 wherein R is selected from the group consisting of hydrogen, methyl, and combinations thereof, m is an integer from 1 to 2000, x is independently zero or greater, y is independently zero or greater, wherein the dimethicone copolyol has at least one ethylene oxide and/or propylene oxide, and has an HLB value of about 20 or less; and
(iii) mixtures thereof; and

4. The hair conditioning composition according to Claim 1 further comprising an additional frizz control agent selected from the group consisting of (i), (ii), and (iii):
(i) polypropylene glycol having a weight average molecular weight of from about 200 g/mol to about 100,000 g/mol; and
(ii) pentaerythritol ester oils having the formula: 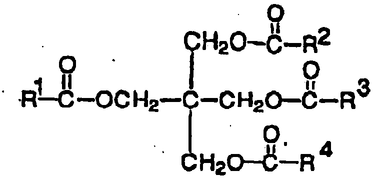 wherein R¹, R², R³, and R⁴, Independently, are branched, straight, saturated, or unsaturated alkyl, aryl, and alkylaryl groups having from 1 to about 30 carbons; and
(iii) mixtures thereof.

5. The hair conditioning composition according to Claim 1 further comprising a silicone compound.

6. The hair conditioning composition according to Claim 5 wherein the silicone compound is selected from the group consisting of silicone gums, silicone resins, and mixtures thereof.

7. The hair conditioning composition according to Claim 1 further comprising a humectant.

8. The hair conditioning composition according to Claim 1 further comprising an amphoteric conditioning polymer.

9. The hair conditioning composition according to Claim 1, wherein the thickening agent is the acrylic acid/alkyl acrylate copolymer.

10. The hair conditioning composition according to Claim 1, wherein the thickening agent is a combination of at least 2 thickening agents selected from the group consisting of (i), (ii), and (iii);
(i) a hydrophobically modified cellulose ether;
(ii) the acrylates copolymer;
(iii) the crosslinked polymer.

11. The hair conditioning composition according to Claim 10 wherein the combination of thickening agents comprises the hydrophobically modified cellulose ether, the acrylates copolymer, and the crosslinked polymer.

12. The hair conditioning composition according to Claim 10 wherein the acrylates copolymer comprises by weight:
(a) from about 5% to about 80% of a C₁-C₈ alkyl ester of acrylic acid;
(b) from about 5% to about 80% of a mono- or di-(C₁-C₄)alkylamino(C₁₋C₄)alkyl(meth)acrytate; and
(c) from about 0.1% to about 10% of a ethylenically unsaturated copolymerizable surfactant monomers obtained by condensing a nonionic surfactant with an acid, wherein the acid is selected from the group consisting of α,β-ethylenically unsaturated carboxylic acids, anhydrides of α,β-ethylanically unsaturated carboxylic acids, and mixtures thereof.

13. The hair conditioning composition according to Claim 10, wherein the crosslinked polymer is a homopolymer comprising the monomer (B).

14. The hair conditioning composition according to Claim 13 wherein the crosslinked polymer is a homopolymer comprising a quaternized dialkylaminoalkyl methacrylate.

15. The hair conditioning composition according to Claim 10 wherein the hydrophobically modified cellulose ether is nonionic, the acrylates copolymer is cationic, and the crosslinked polymer is cationic.

16. The hair conditioning composition according to Claim 10 further comprising a cationic conditioning agent.

17. The hair conditioning composition according to Claim 16 wherein the cationic conditioning agent is a cationic surfactant.

18. The hair conditioning composition according to Claim 1 comprising by weight:
(1) from about 0.05% to about 10% of the thickening agent;
(2) from about 0.1% to about 20% of the frizz control agent;
(3) from about 0.1% to about 40% of a silicone compound;
(4) from about 0.01 % to about 10% of an amphoteric conditioning polymer;
(5) from about 0.1 % to about 20% of a humectant; and
(6) an aqueous carrier.

## Patentansprüche

1. Zum Verbleib im Haar bestimmte Haarkonditionierungszusammensetzung umfassend:
(1) ein Verdickungsmittel ausgewählt aus der Gruppe bestehend aus (i), (ii), (iii) und (iv);
(i) ein Acrylsäure-/Alkylacrylat-Copolymer mit der folgenden Formel: 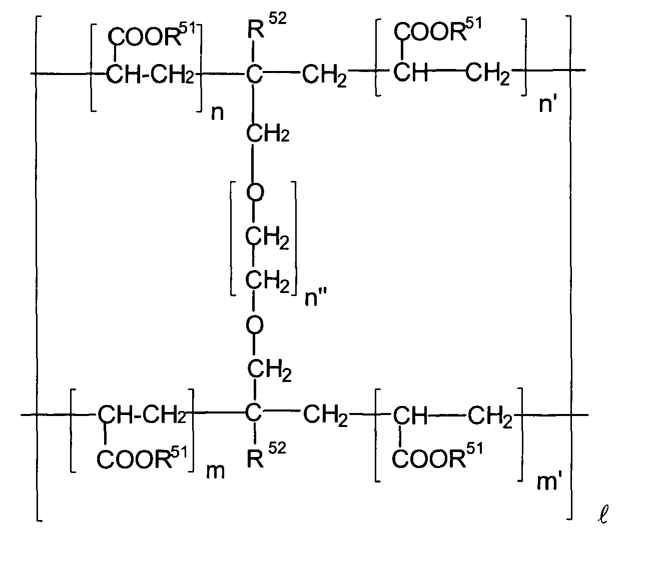 worin R⁵¹ unabhängig ein Wasserstoff oder ein Alkyl mit 1 bis 30 Kohlenstoffen ist, wobei mindestens einer der Reste R⁵¹ ein Wasserstoff ist, R⁵² wie oben definiert lautet, n, n', m und m' ganze Zahlen sind, wobei n+n'+m+m' von etwa 40 bis etwa 100 beträgt, n" eine ganze Zahl von 1 bis etwa 30 ist und ℓ so definiert ist, dass das Copolymer ein Molekulargewicht von etwa 500.000 bis etwa 3.000.000 aufweist.
(ii) ein Acrylat-Copolymer umfassend nach Gewichtsanteilen:
(a) von etwa 5 Gew.-% bis etwa 80 Gew.-% ein Acrylatmonomer ausgewählt aus der Gruppe bestehend aus einem C₁-C₆-Alkylester aus Acrylsäure, einem C₁-C₆-Alkylester aus Methacrylsäure und Mischungen davon;
(b) von etwa 5 Gew.-% bis etwa 80 Gew.-% ein Monomer ausgewählt aus der Gruppe bestehend aus einer vinylsubstituierten heterocyclischen Verbindung, die mindestens ein Stickstoff- oder Schwefelatom, ein (Meth)acrylamid, ein Mono- oder Di-(C₁-C₄)-alkylamino(C₁-C₄)alkyl(meth)acrylat, ein Mono- oder Di-(C₁₋C₄)alkylamino(C₁-C₄)alkyl(meth)acrylamid und Mischungen davon enthält; und
(c) von 0 Gew.-% bis etwa 30 Gew.-% ein assoziatives Monomer;
(iii) ein vernetztes Polymer der Formel (A)ₘ(B)ₙ(C)ₚ, worin:
(A) ausgewählt aus der Gruppe bestehend aus einem Dialkylaminoalkylacrylat, einem quaternisierten Dialkylaminoalkylacrylat, einem Säureadditionssalz eines quaternisierten Dialkylaminoalkylacrylats und Mischungen davon ist;
(B) ausgewählt aus der Gruppe bestehend aus einem Dialkylaminoalkylmethacrylat, einem quaternisierten Dialkylaminoalkylmethacrylat, einem Säureadditionssalz eines quaternisierten Dialkylaminoalkylmethacrylats und Mischungen davon ist;
(C) ein nichtionisches Monomer ist, das mit (A) oder (B) polymerisierbar ist; und m, n und p unabhängig voneinander Null oder größer sind, jedoch mindestens eines von m oder n Eins oder größer beträgt; und
(iv) Mischungen davon.
(2) ein Frizz-Control-Mittel ausgewählt aus der Gruppe bestehend aus (i), (ii), (iii) und (iv);
(i) PEG-modifizierte Glyceride der Struktur: 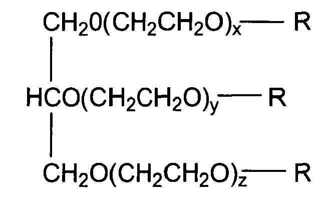 worin ein oder mehrere der R-Gruppen aus gesättigten oder ungesättigten Fettsäureeinheiten ausgewählt sind, die von tierischen oder pflanzlichen Ölen abgeleitet sind, worin die Fettsäureeinheiten eine Kohlenstoffkettenlänge von 12 bis 22 aufweisen, jede beliebigen anderen R-Gruppen sind Wasserstoff, x, y, z betragen unabhängig voneinander Null oder mehr, die durchschnittliche Summe von x+y+z beträgt von etwa 10 bis etwa 45;
(ii) PEG-modifizierte Glycerylfettsäureester mit folgender Struktur: 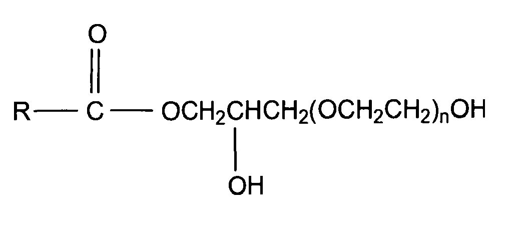 worin R eine aliphatische Gruppe ist, die eine Kohlenstoffkettenlänge von 12 bis 22 aufweist und wobei n einen durchschnittlichen Wert von 15 bis 30 besitzt; und
iii) Mischungen davon; und
(3) einen wässrigen Träger.

2. Die Haarkonditionierungszusammensetzung nach Anspruch 1, worin die Summe aus x+y+z in den PEG-modifizierten Glyceriden von etwa 20 bis 30 beträgt.

3. Die Haarkonditionierungszusammensetzung nach Anspruch 1, ferner umfassend Dimethiconcopolyole ausgewählt aus der Gruppe bestehend aus (i), (ii) und (iii):
(i) Dimethiconcopolyole mit der Struktur: 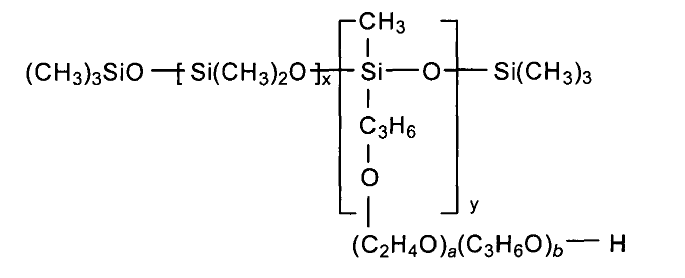 worin x eine ganze Zahl von 1 bis 2.000 ist, y eine ganze Zahl von 1 bis 1.000 ist, a Null oder größer beträgt, b Null oder größer beträgt, die Summe von a+b mindestens 1 ist und ein HLB-Wert von etwa 20 oder weniger vorliegt;
(ii) Dimethiconcopolyole mit der Struktur: 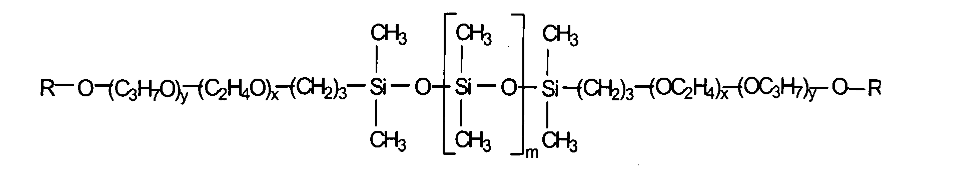 worin R ausgewählt aus der Gruppe bestehend aus Wasserstoff, Methyl und Kombinationen davon ist, m eine ganze Zahl von 1 bis 2.000 ist, x unabhängig voneinander Null oder größer beträgt, y unabhängig voneinander Null oder größer beträgt, worin das Dimethiconcopolyol mindestens ein Ethylenoxid und/oder Propylenoxid aufweist und wobei ein HLB-Wert von etwa 20 oder weniger vorliegt; und
(iii) Mischungen davon; und

4. Die Haarkonditionierungszusammensetzung nach Anspruch 1, ferner umfassend ein weiteres Frizz-Control-Mittel ausgewählt aus der Gruppe bestehend aus (i), (ii) und (iii):
(i) Polypropylenglycol mit einem durchschnittlichen Molekulargewicht von etwa 200 g/mol bis etwa 100.000 g/mol; und
(ii) Pentaerythritesteröle mit der Formel: 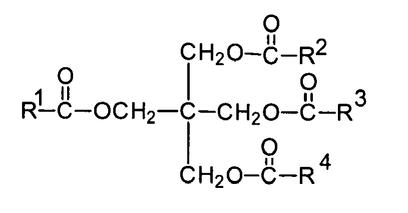 worin R¹, R², R³ und R⁴ unabhängig voneinander verzweigte, gerade, gesättigte oder ungesättigte Alkyl-, Aryl- und Alkylarylgruppen mit 1 bis etwa 30 Kohlenstoffatomen sind; und
(iii) Mischungen davon.

5. Die Haarkonditionierungszusammensetzung nach Anspruch 1, ferner umfassend eine Silikonverbindung.

6. Die Haarkonditionierungszusammensetzung nach Anspruch 5, worin die Silikonverbindung ausgewählt aus der Gruppe bestehend aus Silikonkautschuk, Silikonharzen und Mischungen davon ist.

7. Die Haarkonditionierungszusammensetzung nach Anspruch 1, ferner umfassend eine Feuchthaltemittel.

8. Die Haarkonditionierungszusammensetzung nach Anspruch 1, ferner umfassend ein amphoteres Konditionierungspolymer.

9. Die Haarkonditionierungszusammensetzung nach Anspruch 1, worin das Verdickungsmittel das Acrylsäure-/Alkylacrylatcopolymer ist.

10. Die Haarkonditionierungszusammensetzung nach Anspruch 1, worin das Verdickungsmittel eine Kombination aus mindestens 2 Verdickungsmitteln ausgewählt aus der Gruppe bestehend aus (i), (ii) und (iii) ist;
(i) ein hydrophob modifiziertes Celluloseether;
(ii) das Acrylatcopolymer;
(iii) das vernetzte Polymer.

11. Die Haarkonditionierungszusammensetzung nach Anspruch 10, worin die Kombination der Verdickungsmittel das hydrophob modifizierte Celluloseether, das Acrylatcopolymer und das vernetzte Polymer umfasst.

12. Die Haarkonditionierungszusammensetzung nach Anspruch 10, worin das Acrylatcopolymer nach Gewichtsanteilen Folgendes umfasst:
(a) von etwa 5 Gew.-% bis etwa 80 Gew.-% ein C₁-C₆-Alkylester aus Acrylsäure;
(b) von etwa 5 Gew.-% bis etwa 80 Gew.-% ein Mono- oder Di-(C₁-C₄)-alkylamino(C₁-C₄)alkyl(meth)acrylat; und
(c) von etwa 0,1 Gew.-% bis etwa 10 Gew.-% ein ethylenisch ungesättigtes copolymerisierbares Tensidmonomer, das durch die Kondensation eines nichtionischen Tensids mit einer Säure hergestellt wurde, worin die Säure ausgewählt aus der Gruppe bestehend aus α,β-ethylenisch ungesättigten Carbonsäuren, Anhydriden aus α,β-ethylenisch ungesättigten Carbonsäuren und Mischungen davon ist.

13. Die Haarkonditionierungszusammensetzung nach Anspruch 10, worin das vernetzte Polymer ein Homopolymer umfassend das Monomer (B) ist.

14. Die Haarkonditionierungszusammensetzung nach Anspruch 13, worin das vernetzte Polymer ein Homopolymer, umfassend ein quaternisiertes Dialkylaminoalkylmethacrylat, ist.

15. Die Haarkonditionierungszusammensetzung nach Anspruch 10, worin das hydrophob modifizierte Celluloseether nichtionisch ist, das Acrylatcopolymer kationisch ist und das vernetzte Polymer kationisch ist.

16. Die Haarkonditionierungszusammensetzung nach Anspruch 10, ferner umfassend ein kationisches Konditioniermittel.

17. Die Haarkonditionierungszusammensetzung nach Anspruch 16, worin das kationische Konditioniermittel ein kationisches Tensid ist.

18. Die Haarkonditionierungszusammensetzung nach Anspruch 1, ferner umfassend nach Gewichtsanteilen:
(1) von etwa 0,05 Gew.-% bis etwa 10 Gew.-% das Verdickungsmittel;
(2) von etwa 0,1 Gew.-% bis etwa 20 Gew.-% das Frizz-Control-Mittel;
(3) von etwa 0,1 Gew.-% bis etwa 40 Gew.-% eine Silikonverbindung;
(4) von etwa 0,01 Gew.-% bis etwa 10 Gew.-% ein amphoteres Konditionierpolymer;
(5) von etwa 0,1 Gew.-% bis etwa 20 Gew.-% ein Feuchthaltemittel; und
(6) einen wässrigen Träger.

## Revendications

1. Composition de conditionnement des cheveux pour utilisation « à laisser » sur les cheveux comprenant :
(1) un agent épaississant choisi dans le groupe constitué de (i), (ii), (iii), et (iv) ;
(i) un copolymère d'acide acrylique/alkyl-acrylate ayant la formule suivante : 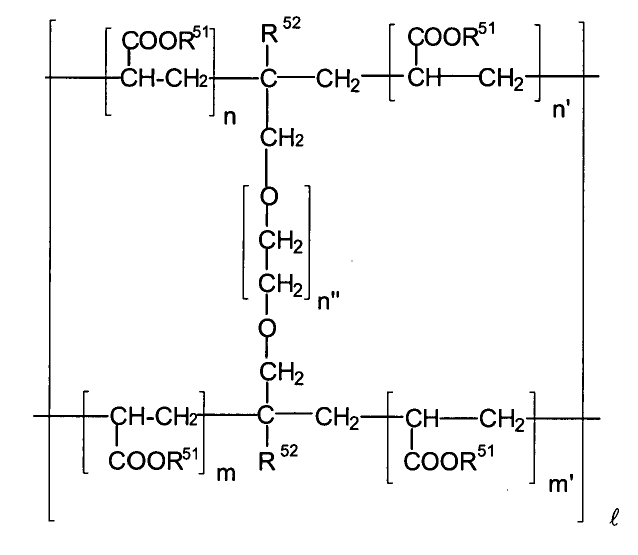 dans laquelle R⁵¹ est indépendamment un hydrogène ou un alkyle de 1 à 30 carbones dans lequel au moins un des R⁵¹ est un hydrogène, R⁵² est tel que défini ci-dessus, n, n', m et m' sont des entiers tels que n+n'+m+m' est d'environ 40 à environ 100, n" est un entier allant de 1 à environ 30, et ℓ est défini de sorte que le copolymère ait un poids moléculaire d'environ 500 000 à environ 3 000 000.
(ii) un copolymère acrylate comprenant en poids :
(a) d'environ 5 % à environ 80 % d'un monomère acrylate choisi dans le groupe constitué d'un alkylester d'acide acrylique en C₁ à C₆, un alkylester en C₁ à C₆ d'acide méthacrylique, et leurs mélanges ;
(b) d'environ 5 % à environ 80 % d'un monomère choisi dans le groupe constitué d'un composé hétérocyclique vinyle-substitué contenant au moins un atome d'azote ou de soufre, un (méth)acrylamide, un mono- ou di-(C₁ à C₄)alkylamino(C₁ à C₄)alkyl(méth)acrylate, un mono- ou di-(C₁ à C₄)alkylamino(C₁ à C₄)alkyl(méth)acrylamide, et leurs mélanges ; et
(c) de 0 % à environ 30 % d'un monomère associatif ;
(iii) un polymère réticulé de formule (A)ₘ(B)ₙ(C)ₚ, dans lequel :
(A) est choisi dans le groupe constitué d'un acrylate de dialkylaminoalkyle, un acrylate de dialkylaminoalkyle quaternaire, un sel d'addition acide d'un acrylate de dialkylaminoalkyle quaternaire, et leurs mélanges ;
(B) est choisi dans le groupe constitué d'un méthacrylate de dialkylaminoalkyle, un méthacrylate de dialkylaminoalkyle quaternaire, un sel d'addition acide d'un méthacrylate de dialkylaminoalkyle quaternaire, et leurs mélanges ;
(C) est un monomère non ionique polymérisable avec (A) ou (B) ; et m, n, et p sont indépendamment zéro ou plus, mais au moins un des m ou n est un ou plus grand ; et
(iv) leurs mélanges ;
(2) un agent de contrôle de frisure choisi dans le groupe constitué de (i), (ii), et (iii) :
(i) des glycérides modifiés par du PEG de structure : 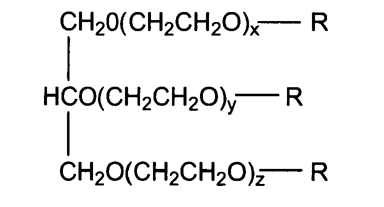 dans laquelle un ou plusieurs des groupes R sont choisis parmi les fragments d'acide gras saturés ou insaturés dérivés d'huiles animales ou végétales dans lesquelles les fragments d'acide gras ont une longueur de chaîne carbonée comprise entre 12 et 22, n'importe quels autres groupes R sont un hydrogène, x, y, z sont indépendamment zéro ou plus, la somme moyenne de x+y+z est égale à d'environ 10 à environ 45 ;
(ii) des esters d'acide gras glycéryle modifiés par du PEG de structure : 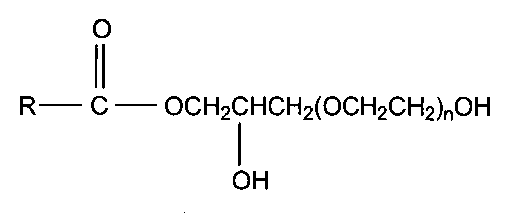 dans laquelle R est un groupe aliphatique ayant une longueur de chaîne carbonée de 12 à 22, et n a une valeur moyenne allant de 15 à 30 ; et
(iii) leurs mélanges ; et
(3) un véhicule aqueux.

2. Composition de conditionnement des cheveux selon la revendication 1, dans laquelle la somme de x+y+z dans les glycérides modifiés par du PEG est égale à d'environ 20 à 30.

3. Composition de conditionnement des cheveux selon la revendication 1, comprenant en outre des copolyols de diméthicone choisis dans le groupe constitué de (i), (ii), et (iii) :
(i) des copolyols de diméthicone de structure : 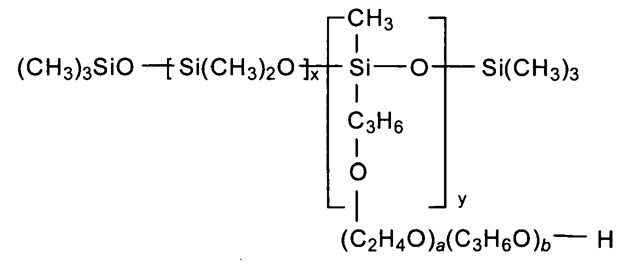 dans laquelle x est un entier de 1 à 2000, y est un entier de 1 à 1000, a est zéro ou plus grand, b est zéro ou plus grand, la somme de a+b est au moins 1, et ayant un rapport hydro-lipophile d'environ 20 ou moins ;
(ii) des copolyols de diméthicone de structure : 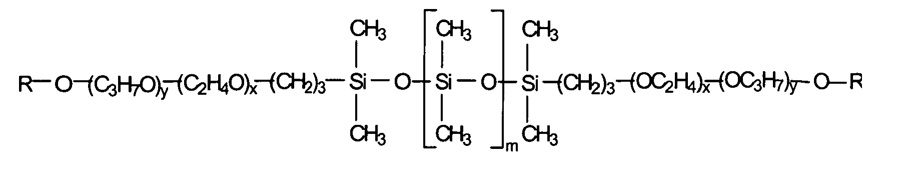 dans laquelle R est choisi dans le groupe constitué d'hydrogène, méthyle, et leurs combinaisons, m est un entier de 1 à 2000, x est indépendamment zéro ou plus grand, y est indépendamment zéro ou plus grand, dans laquelle le copolyol de diméthicone a au moins un oxyde d'éthylène et/ou un oxyde de propylène, et a un rapport hydro-lipophile d'environ 20 ou moins ; et
(iii) leurs mélanges ; et

4. Composition de conditionnement des cheveux selon la revendication 1, comprenant en outre un agent de contrôle de frisure supplémentaire choisi dans le groupe constitué de (i), (ii), et (iii) :
(i) un polypropylène glycol ayant une masse moléculaire moyenne en poids allant d'environ 200 g/mol à environ 100 000 g/mol ; et
(ii) des huiles d'ester de penta-érythritol de formule : 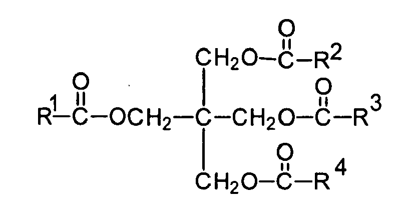 dans laquelle R¹, R², R³, et R⁴, indépendamment, sont des groupes alkyle, aryle, et alkylaryle ramifiés, linéaires, saturés, ou insaturés, ayant de 1 à environ 30 carbones ; et
(iii) leurs mélanges.

5. Composition de conditionnement des cheveux selon la revendication 1, comprenant en outre un composé de silicone.

6. Composition de conditionnement des cheveux selon la revendication 5, dans laquelle le composé de silicone est choisi dans le groupe constitué de gommes de silicone, résines de silicone, et leurs mélanges.

7. Composition de conditionnement des cheveux selon la revendication 1, comprenant en outre un humidifiant.

8. Composition de conditionnement des cheveux selon la revendication 1, comprenant en outre un polymère de conditionnement amphotère.

9. Composition de conditionnement des cheveux selon la revendication 1, dans laquelle l'agent épaississant est le copolymère acide acrylique/acrylate d'alkyle.

10. Composition de conditionnement des cheveux selon la revendication 1, dans laquelle l'agent épaississant est une combinaison d'au moins 2 agents épaississants choisis dans le groupe constitué de (i), (ii), et (iii) ;
(i) un éther de cellulose « rendu hydrophobe»;
(ii) le copolymère d'acrylates ;
(iii) le polymère réticulé.

11. Composition de conditionnement des cheveux selon la revendication 10, dans laquelle la combinaison d'agents épaississants comprend l'éther de cellulose « rendu hydrophobe », le copolymère d'acrylates, et le polymère réticulé.

12. Composition de conditionnement des cheveux selon la revendication 10, dans laquelle le copolymère d'acrylates comprend en poids :
(a) d'environ 5 % à environ 80 % d'un alkylester en C₁ à C₆ d'acide acrylique ;
(b) d'environ 5 % à environ 80 % d'un mono- ou di-(C₁ à C₄)alkylamino(C₁ à C₄)alkyle(méth)acrylate ; et
(c) d'environ 0,1 % à environ 10 % de monomères d'agent tensioactif copolymérisables éthyléniquement insaturés obtenus en condensant un agent tensioactif non ionique avec un acide, dans lequel l'acide est choisi dans le groupe constitué d'acides carboxyliques éthyléniquement insaturés α,β, d'anhydrides d'acides carboxyliques éthyléniquement insaturés α,β, et leurs mélanges.

13. Composition de conditionnement des cheveux selon la revendication 10, dans laquelle le polymère réticulé est un homopolymère comprenant le monomère (B).

14. Composition de conditionnement des cheveux selon la revendication 13, dans laquelle le polymère réticulé est un homopolymère comprenant un méthacrylate de dialkylaminoalkyle quaternaire.

15. Composition de conditionnement des cheveux selon la revendication 10, dans laquelle l'éther de cellulose « rendu hydrophobe » est non ionique, le copolymère d'acrylates est cationique, et le polymère réticulé est cationique.

16. Composition de conditionnement des cheveux selon la revendication 10, comprenant en outre un agent de conditionnement cationique.

17. Composition de conditionnement des cheveux selon la revendication 16, dans laquelle l'agent de conditionnement cationique est un agent tensioactif cationique.

18. Composition de conditionnement des cheveux selon la revendication 1, comprenant en poids :
(1) d'environ 0,05 % à environ 10 % de l'agent épaississant ;
(2) d'environ 0,1% à environ 20 % de l'agent de contrôle de frisure ;
(3) d'environ 0,1% à environ 40 % d'un composé de silicone ;
(4) d'environ 0,01% à environ 10 % d'un polymère de conditionnement amphotère ;
(5) d'environ 0,1% à environ 20 % d'un humidifiant ; et
(6) un véhicule aqueux.
